# EUROPÄISCHE PATENTSCHRIFT

(11) **EP 1 139 973 B1**
(45) Veröffentlichungstag und Bekanntmachung des Hinweises auf die Patenterteilung: **23.04.2003**
(21) Anmeldenummer: 99969226.2
(22) Anmeldetag: 22.12.1999
(51) Int. Cl.: C07F 7/21, A61K 6/093

(54) **POLYMERISIERBARE MASSEN AUF DER BASIS VON ZUR AUSHÄRTUNG BEFÄHIGTEN SILOXANVERBINDUNGEN**
POLYMERISABLE MATERIALS WHICH ARE BASED ON HARDENABLE SILOXANE COMPOUNDS
MATIERES POLYMERISABLES A BASE DE COMPOSES DE SILOXANE DURCISSABLES

(30) Priorität: 24.12.1998 DE 19860364
(43) Veröffentlichungstag der Anmeldung: 10.10.2001
(73) Patentinhaber: 3M Espe AG, 82229 Seefeld (DE)
(72) Erfinder: WEINMANN, Wolfgang, D-82205 Gilching (DE); ZECH, Joachim, D-82229 Seefeld (DE); GASSER, Oswald, D-82229 Seefeld (DE); GUGGENBERGER, Rainer, D-82211 Herrsching (DE); ECKHARDT, Gunther, D-06231 Bad Dürrenberg (DE); BISSINGER, Peter, D-86911 Diessen (DE); SOGLOWEK, Wolfgang, D-86911 Die en-Obermühlhausen (DE)
(74) Vertreter: Freiherr von Wittgenstein, Arved, Dr.
(86) Internationale Anmeldenummer: EP9910319
(87) Internationale Veröffentlichungsnummer: WO00038619

(56) Entgegenhaltungen:
- EP-A- 0 261 520
- EP-A- 0 866 086
- DE-A- 19 648 283
- DE-A- 19 736 665
- GB-A- 2 023 628
- GB-A- 2 086 914

## Beschreibung

Die vorliegende Erfindung betrifft polymerisierbare Massen für zahnärztliche und zahntechnische Anwendungen auf der Basis von zur Aushärtung befähigten Siloxanverbindungen.

In polymerisierbaren Dentalmassen werden bislang vorwiegend ethylenisch ungesättigte Monomere, bevorzugt Methacrylat- und Acrylatmonomere verwendet.

Besonders häufig wird dabei das von Bowen beschriebene 2,2-Bis[4,1-phenylenoxy(2-hydroxy-3,1-propandiyl)-methacryisäureester]-propyliden (Bis-GMA) [US-A-3 066 112] eingesetzt. Mischungen dieses Methacrylats mit Triethylenglykoldimethacrylat (TEGDMA) dienen auch heute noch als Standardrezeptur für dentale plastische Direkt-Füllungswerkstoffe. Auch Methacrylderivate des zweifach formylierten Bis-(hydroxymethyl)tricyclo[5.2.1.0^{2,6}]-decans haben sich als Monomere für Dentalcomposite bewährt [W. Gruber et al., DE-A-27 14 538; W. Schmitt et al., DE-C-28 16 823; J. Reiners et al., EP-A-0 261 520].

All diesen Zusammensetzung gemeinsam ist der bei der Polymerisation auftretende nachteilige Polymerisationsschrumpf. Dieser kann beispielsweise bei der Anwendung als Füllungsmaterial zur Bildung von Verfärbungen am Kavitätenrand des Zahnes oder sogar zur Entstehung von Randspalten mit anschließendem Sekundärkariesrisiko führen.

In der Vergangenheit hat man deshalb versucht, den Polymerisationsschrumpf der Dentalmassen durch einen möglichst hohen Anteil an anorganischen Füllkörpern zu vermindern. Dies führt aber in aller Regel zu einer deutlichen Erhöhung der Viskosität solcher Massen mit Handlingsnachteilen für den Anwender, der dann zur Verarbeitung dieser Materialien gegebenenfalls zu Hilfsmitteln wie z.B. Ultraschall greifen muss [EP-0 480 472].

Üblicherweise verwendete Dentalmonomere beinhalten meist eine oder maximal zwei polymerisierbare Gruppen. Eine höhere Funktionalität an radikalisch vernetzenden Gruppen pro Molekül führt in der Regel zu sehr hochviskosen Substanzen, die sich nur schwer mit Füllstoffen vermengen lassen und zu sehr spröden Materialien im ausgehärteten Zustand führen.

Niedrig funktionalisierte Monomere haben jedoch den Nachteil, daß sie wenige Verknüpfungspunkte zur Vernetzung anbieten und deshalb bei nicht vollständiger Polymerisation aller Monomere (welche praktisch nie zu 100% erreicht wird) auch nach der Aushärtungsreaktion noch als Monomere vorliegen. Diese sog Restmonomere können über einen längeren Zeitraum hinweg aus dem Dentalmaterial herausgelöst werden und zu unerwünschten Nebenwirkungen im Organismus führen.

Aus der GB-A-2 086 914 sind Zusammensetzungen auf der Basis eines monofunktionellen Methylmethacrylat-Monomeren bekannt, welches 0,5 bis 20 Gew.-% einer Silanverbindung mit hydrolisierbaren Gruppen enthält. Die hydrolisierbaren Silangruppen dienen zum Andocken an die Zahnsubstanz, um auf diese Weise eine verbesserte Verbindung zwischen den Basismonomeren und der Zahnsubstanz herzustellen. Es handelt sich nicht um polymere -Si-O-Si-O- Verbindungen.

Die DE-A-126 48 283 betrifft polymerisierbare Massen auf der Basis bestimmter Epoxide. Die ringförmigen Epoxidgruppen befinden sich dabei jeweils am Ende der Moleküle. Die Polymerisation solcher Systeme läuft verhältnismäßig langsam ab und ist ihrer Natur nach eine kationische Polymerisation. Sie führt in der Regel zu keinen langen Ketten.

Die EP-A-0 866 086 beschreibt Carbosilan-Dendrimere und deren Herstellung. Diese Verbindungen eignen sich zur Herstellung von organischanorganischen Hybridmaterialien und Lacken. Die Verwendung derartiger Verbindungen im Dentalbereich wird nicht angesprochen.

Die GB-A-2 023 628 beschreibt Methacryl-substituierte Alkylsiloxane und deren Verwendung in der Zahnheilkunde. Diese Verbindungen weisen mindestens zwei durch ein einzelnes O-Atom gebundene Cyclosiloxan-Einheiten auf und unterscheiden sich damit von den erfindungsgemäßen Verbindungen.

Die bereits oben erwähnte EP-A-0 261 520 beschreibt lineare Siloxane auf der Basis von (Meth)acrylsäureestern mit Tricyclodecangruppen. Diese Verbindungen enthalten am einen Ende eine Siliciumverbindung und am anderen Ende eine oder mehrere (Meth)acrylsäuregruppen. Die Polymerisation dieser Verbindungen verläuft über einen radikalischen Mechanismus. Diese Monomere eignen sich für die Herstellung von Dentalmassen. Grundsätzlich nachteilig ist bei diesen Monomeren, daß bei zunehmender Dichte an zur Polymerisation befähigten Gruppen die Viskosität und auch der Polymerisationsschrumpf von daraus hergestellten Massen zunehmen. Auch die Füllstoffaufnahme ist nicht zufriedenstellend.

Aufgabe der vorliegenden Erfindung war es daher, Monomere für Dentalmassen bereitzustellen, welche trotz einer hohen Dichte an zur Polymerisation befähigten Gruppen eine niedrige Viskosität aufweisen, eine hohe Füllstoffaufnahme ermöglichen und zu Massen mit geringem Polymerisationsschrumpf führen.

Gelöst wurde die Aufgabe durch die Bereitstellung von neuen Monomeren der folgenden allgemeinen Formel (I): 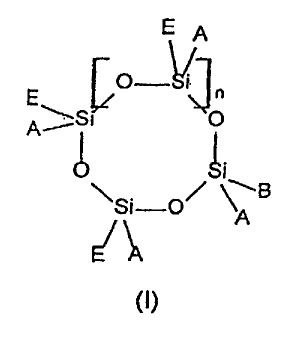 worin bedeuten:
- n =: 0,1,2,3,4,5,6,7,8,9,10 bevorzugt 1,2,3,4,5;
- A =: H oder C₁-C₁₅-Alk(en)yl, bevorzugt Methyl, Ethyl, Propyl, Butyl, Vinyl, Ethinyl, Allyl, C₃-C₁₅-Cycloalk(en)yl, bevorzugt Cyclopentyl, Cyclohexyl, Cyclopentadienyl, Cyclohexenyl, C₈-C₁₂-Aryl, bevorzugt Phenyl, Tolyl, Xylyl, C₈-C₁₈-Alkaryl, bevorzugt Phenylethylenyl, wobei in den genannten Resten jeweils ein oder mehrere C-Atome durch O, C=O, O(C=O), SiR₂ und / oder NR ersetzt sein können, wobei R ein aliphatischer Rest mit 1 bis 7 C-Atomen ist, bei dem ein oder mehrere C-Atome durch O, C=O und / oder O(C=O) ersetzt sein können;
- B=: E oder ein linearer, verzweigter oder polycyclischer aliphatische oder aromatische Gruppen enthaltender Kohlenwasserstoffrest ist, der 2 bis 10, bevorzugt 2 bis 5 oben definierten Cyclosiloxanreste, abzüglich B, miteinander verknüpft und 2 bis 50, bevorzugt 2 bis 30 C-Atome und zusätzlich 0 bis 30, bevorzugt 0 bis 20 andere Atome aus der Gruppe O, N, S, P, Si, Cl, F, Br, I enthält und an welchem entsprechend 1 bis 9, vorzugsweise 1 bis 4 der oben definierten Cydosiloxanreste, abzüglich B, hängen; besonders bevorzugte Reste B sind: Di(prop-3-yl)ether, Di(prop-3-yl)sulfid, Di(prop-3-yl)amin, Di(prop-3-yl)-methyl-amin, Tri(prop-3-yl)amin, Di(prop-3-yl)harnstoff, Di(prop-3-yl)carbonat, Ethylenglykoldi(prop-3-yl)carbonat, Diethylenglykoldi(prop-3-yl)carbonat, Ethylenglykol-di(prop-3-yl)ether, Diethylenglykoldi(prop-3-yl)ether, 1,2-Propandioldi(prop-3-yl)ether, 1,3-Propandioldi(prop-3-yl)ether, 1,3-Butandioldi(prop-3-yl)ether, 1,4-Butandioldi(prop-3-yl)ether, 1,4-Butendioldi(prop-3-yl)ether, 1,4-Butindioldi(prop-3-yl)ether, 1,5-Pentandioldi(prop-3-yl)ether, 1,6-Hexandioldi(prop-3-yl)ether, 1,8-Octandioldi(prop-3-yl)ether, 1,9-Nonandioldi(prop-3-yl)ether, 1,10-Decandioldi(prop-3-yl)ether, 1,12-Dodecandioldi(prop-3-yl)ether, Oxalsäuredi(prop-3-yl)ester, Malonsäuredi(prop-3-yl)ester, Bernsteinsäuredi(prop-3-yl)ester, Adipinsäuredi(prop-3-yl)ether, Sebacinsäuredi(prop-3-yl)ether, 1,2-Ethandiyl, 1,4-Pentadienyl, 1,5-Pentandiyl, 1,5-Hexadienyl, 1,6-Heptadienyl, 1,7-Octadienyl, 1,8-Nonadienyl, 1,9-Decadienyl, 1,11-Dodecadienyl, p-Di(eth-2-yl)benzol, Bis-(4-(prop-3-yl)oxyphenyl)-sulfon, Bis-(4-(prop-3-yl)oxyphenyl)-keton, Bis-(4-(prop-3-yl)oxyphenyl)-methan, 1,1-Bis-(4-(prop-3-yl)oxyphenyl)-ethan, 2,2-Bis-(4-(prop-3-yl)oxyphenyl)-propan, 2,2-Bis-(4-(prop-3-yl)oxyphenyl)-perfluorpropan, 2,2-Bis-(4-(prop-3-yl)oxy-3,5-dibrom-phenyl)-propan, 3,3-Bis-(4-(prop-3-yl)oxyphenyl)-pentan, 4,4-Bis-(4-(prop-3-yl)oxyphenyl)-heptan, 1,1-Bis-(4-(prop-3-yl)oxyphenyl)-cyclopentan, 1,1-Bis-(4-(prop-3-yl)oxyphenyl)-cyclohexan, 1,1-Bis-(4-(prop-3-yl)oxyphenyl)-3,3,5-trimethylcyclohexan, 1,1,1-Tris-(4-(prop-3-yl)oxyphenyl)-ethan, Bis-((prop-3-yl-ether)oxy)-tricyclo[5.2.1.0^{2,6}] decan;
- E =: A oder eine polymerisierbare Gruppe G-Q-L, wobei durchschnittlich bis zu 25% oder weniger der Gruppen E Vertretern von A entsprechen;
- G =: C₁-C₁₀-Alk(en)ylen, bevorzugt Ethylen, Methylethylen, Propylen, Butylen, Hexylen, Ethenylen, Propenylen;
- Q =: O, N-A oder ein zwei- oder mehrwertiger linearer, verzweigter oder cyclischer Alkohol-, Amin- oder Aminoalkohol-Rest mit 2 bis 10 C-Atomen, bevorzugt Ethandiol-diyl, Glycerin-triyl, Trimethylolpropan-triyl, Pentaerythrit-tetryl;
- L =: ein eine C=C-Doppelbindung enthaltender organischer Rest mit 2 bis 10 C-Atomen, bevorzugt Acryl oder Methacryl.

Verbindungen gemäß Formel (I) sind cyclische Siloxane, wobei ein oder mehrere Siloxan-Ringe pro Molekül vorkommen können. Ausdrücklich ausgeschlossen sind jedoch solche Verbindungen, bei denen annelierte Siloxan-Ringsysteme vorliegen.

Die Herstellung von Verbindungen der allgemeinen Formel (I) geschieht durch geeignete Methoden.

Insbesondere durch Hydrosilylierung lassen sich Si-H-funktionelle Cyclosiloxane mit C-C-ungesättigten organischen Gerüsten verknüpfen (B. Marciniec: Comprehensive Handbook on Hydrosilylation, Pergamon Press, 1992). Auf diese Weise lassen sich sowohl die Monomer- als auch die Polymereigenschaften in gewünschter Weise einstellen.

Beispielsweise läßt sich 1,3,5,7-Tetramethylcyclotetrasiloxan in einem Lösungsmittel, wie Toluol, unter Einfluß von Edelmetallkatalysatoren, wie Speir-Katalysator aber auch Wilkinson-Katalysator, mit vier Mol Allyl-methacrylat zu einem Vertreter von (I) verknüpfen. Anstelle des Cyclotetrasiloxans läßt sich auch ein kommerziell verfügbares SiH-Cyclengemisch (ein Gemisch von (SiMeHO)ₙ mit n vorzugsweise 4, 5, 6) verwenden. Statt Allylmethacrylat können andere Allylether, -ester oder -amide von (Meth)acryl-funktionellen organischen Molekülen zum Einsatz kommen.

Bei der Umsetzung von polyfunktionellen SiH-Cyclosiloxanen mit ebenfalls mehrfach C-C ungesättigten organischen Gerüsten können durch geeignete Reaktionsführung alle C-C ungesättigten Funktionen des organischen Gerüstes mit jeweils einem Cyclosiloxanring absättigen. Durch davon abweichende Wahl der Stöchiometrie oder Reaktionsführung lassen sich jedoch auch vorvernetzte Zwischenprodukte herstellen.

Gemeint sind hier beide Möglichkeiten.

Diese SiH-funktionellen Vorstufen lassen sich beispielsweise mit Allylmethacrylat zu weiteren Vertretern von (I) umsetzen.

Die im folgenden aufgezeigten besonders bevorzugten Strukturen werden im Regelfall durch Hydrosilylierung von Allyl- oder Vinyl-Verbindungen mit SiH-haltigen Verbindungen erhalten. Bei einer solchen Hydrosilylierungsreaktion, beispielsweise einer Allyl-Gruppe mit Si-H entstehen in wechselndem Umfange n-Propylen- (β-Addukt) und Methylethylen-Brücken (α-Addukt), jenachdem ob die Si-H-Funktion gegen oder nach der Markovnikov-Regel addiert wird. Die unten aufgeführten besonders bevorzugten Strukturen zeigen im Formelbild nur die n-Propylen (β-Addukt )-Addukte. Gemeint sind jedoch sämtliche möglichen Gemische aus α-und β-Addukt, die bei der Umsetzung immer im Gemisch anfallen (siehe folgendes Schema).

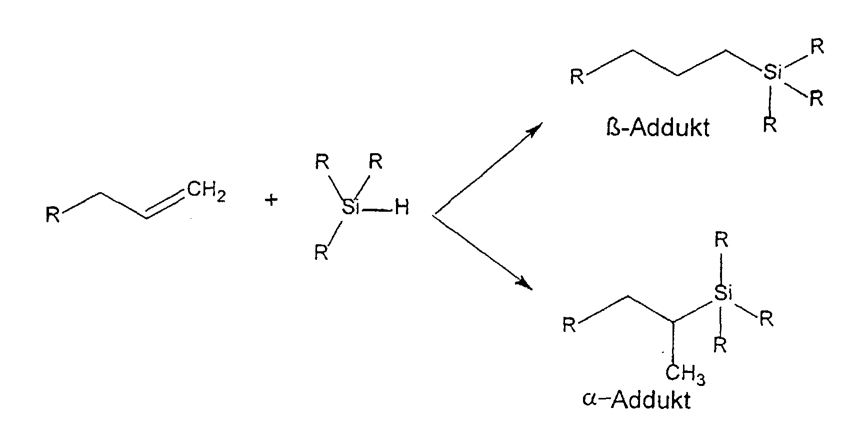

Es wurde überraschenderweise festgestellt, daß diese Verbindungen trotz ihrer hohen Dichte an Acrylat- bzw. Methacrylatgruppen eine sehr geringe Viskosität aufweisen und sich in hervorragender Weise für den Einsatz in Dentalmassen eignen.

Besonders bevorzugte Vertreter der Verbindungen gemäß der allgemeinen Formel (I) sind:

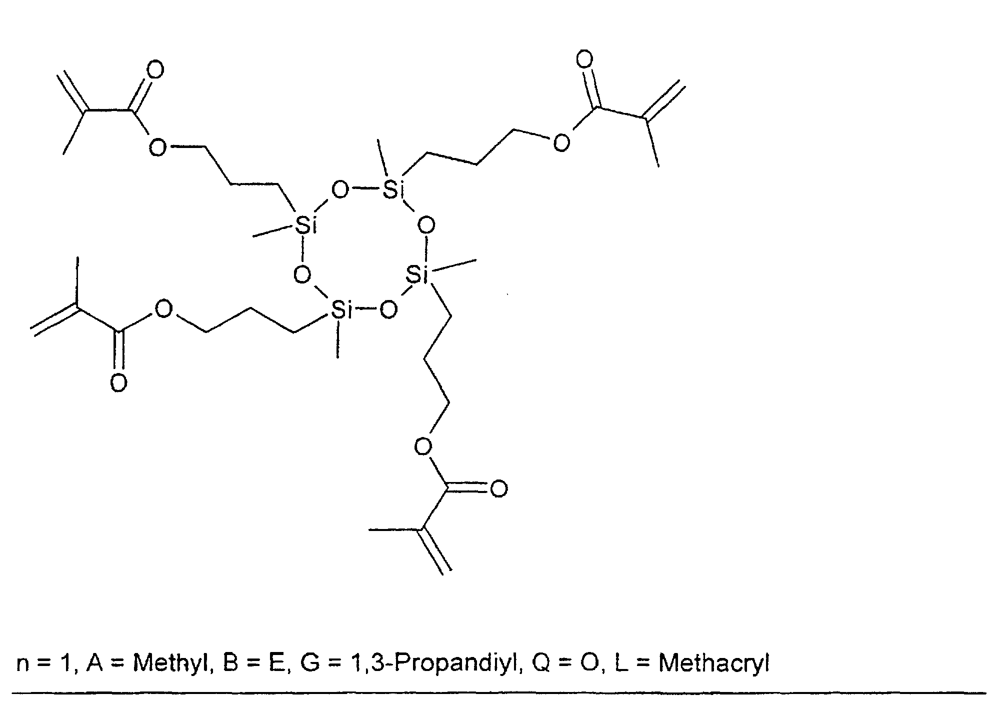

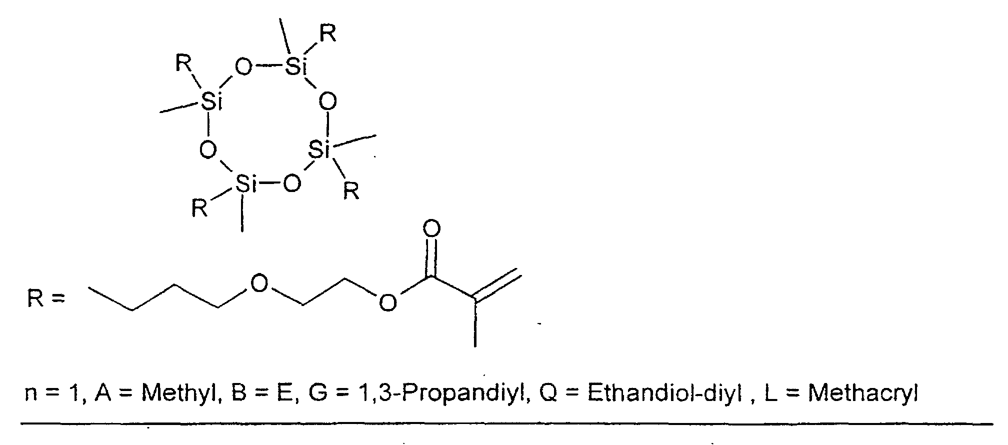

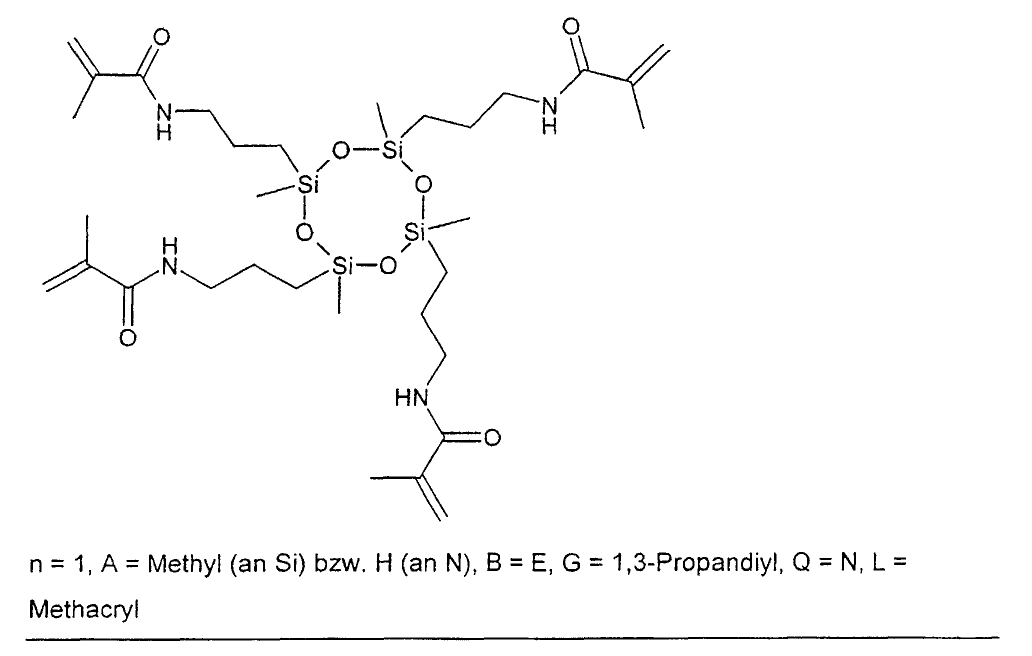

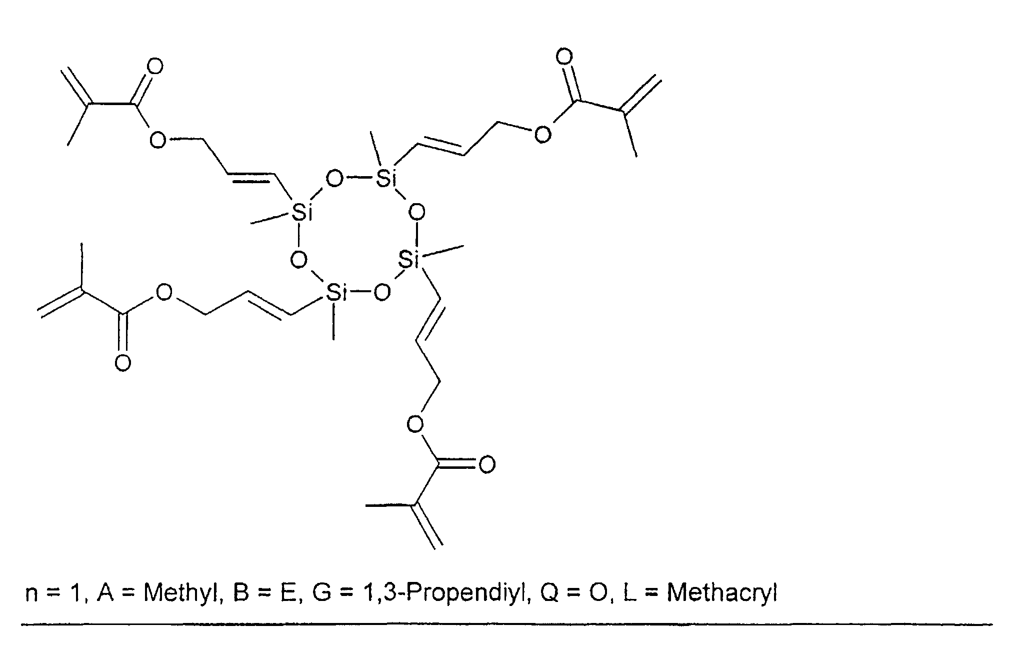

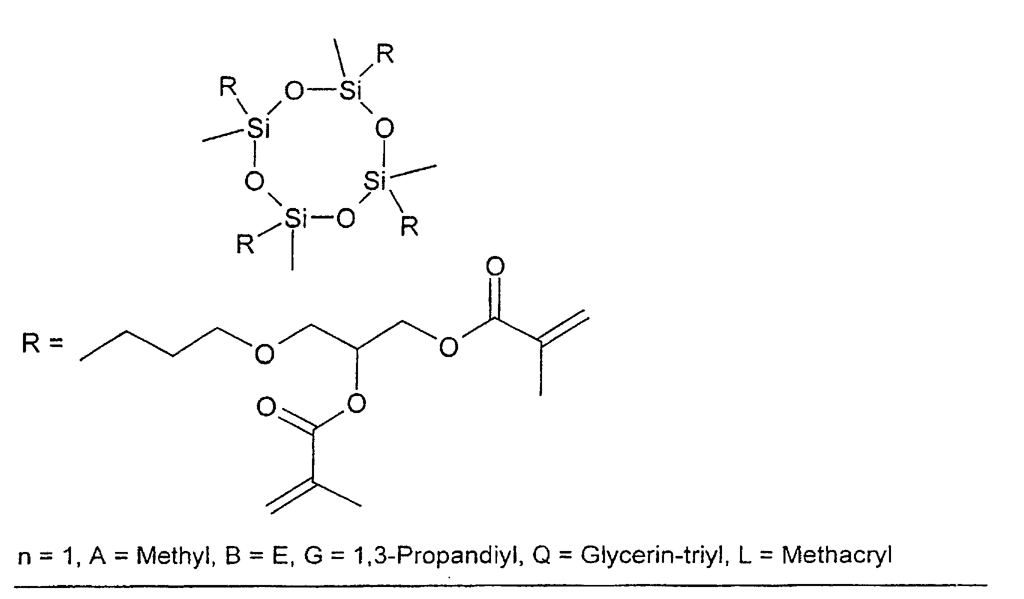

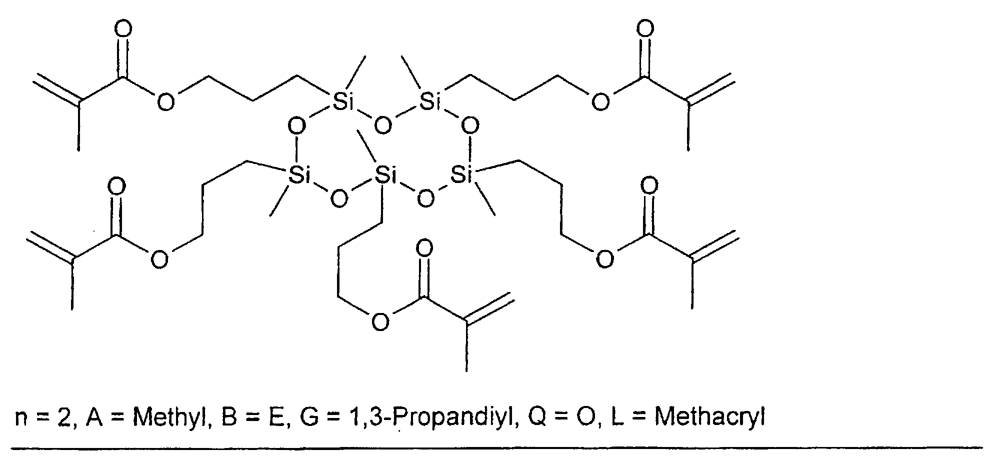

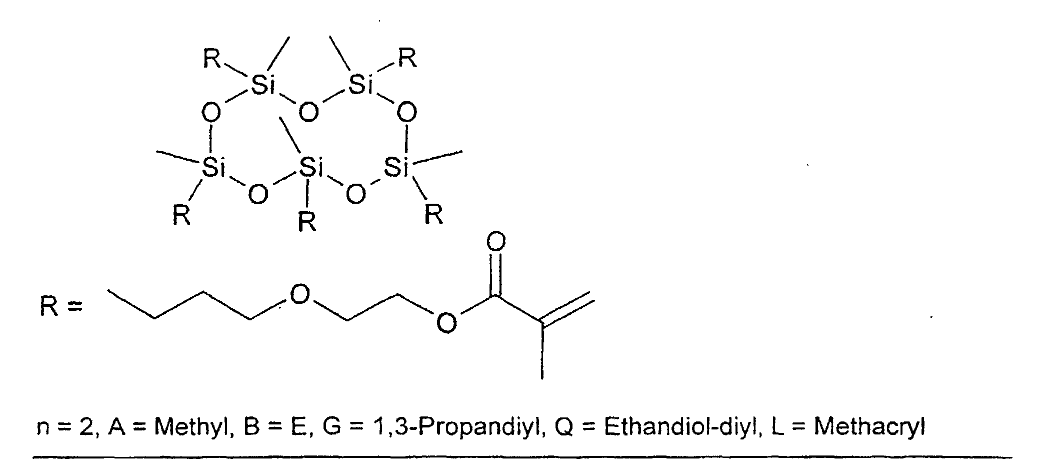

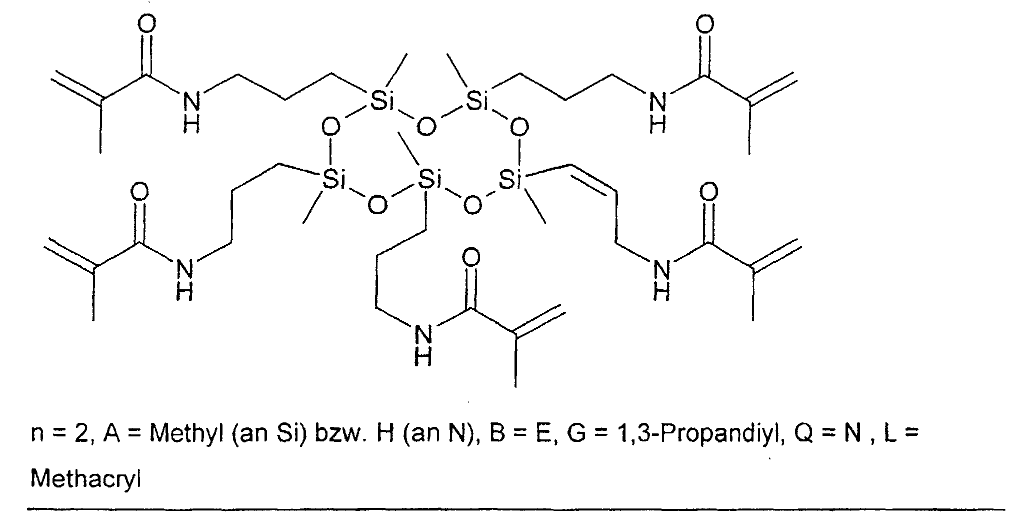

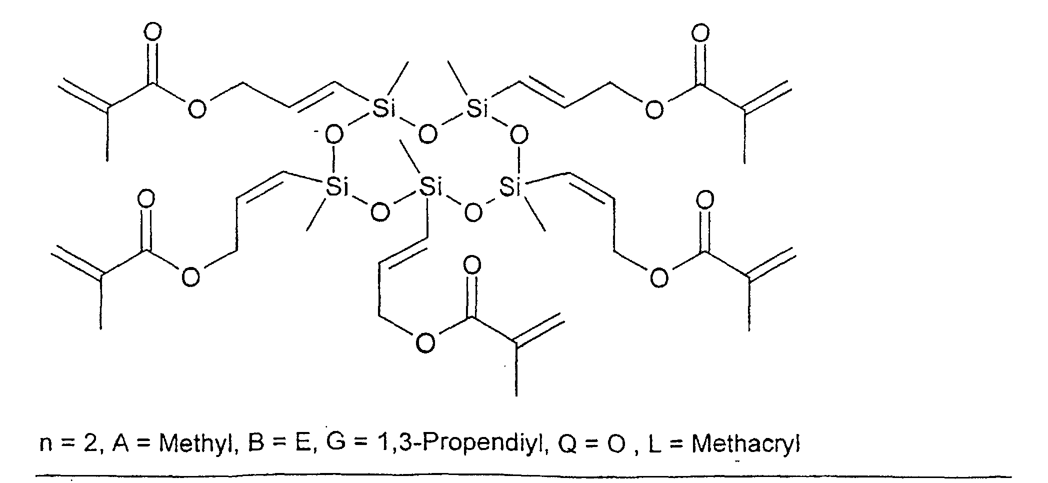

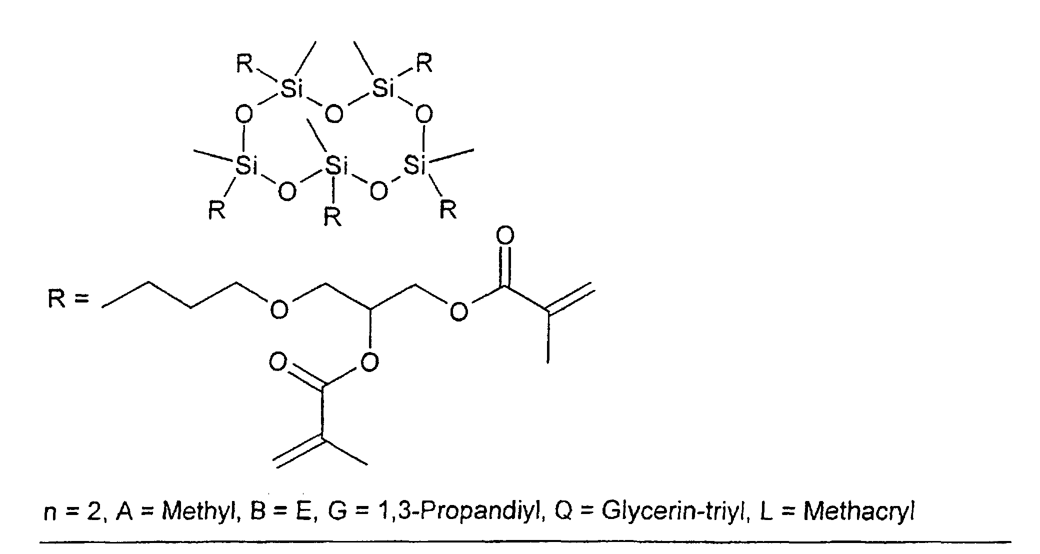

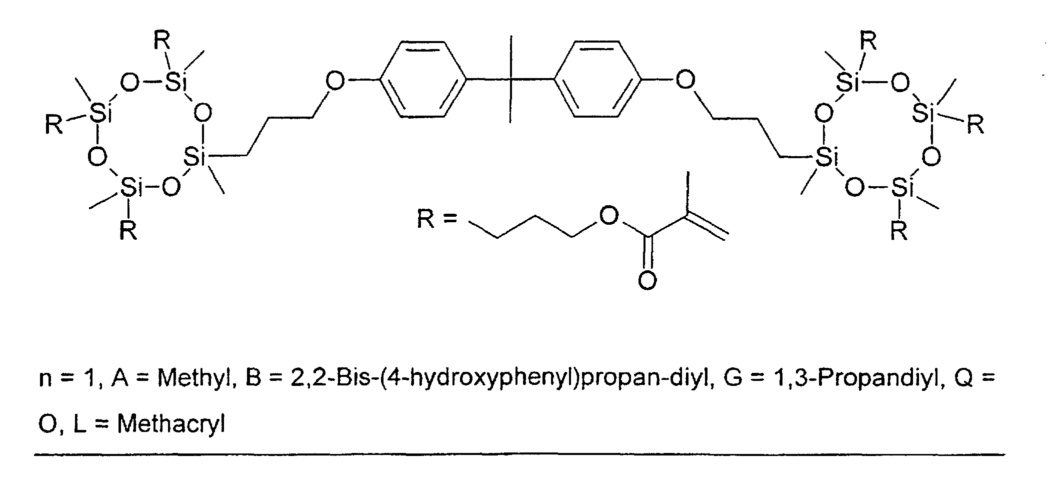

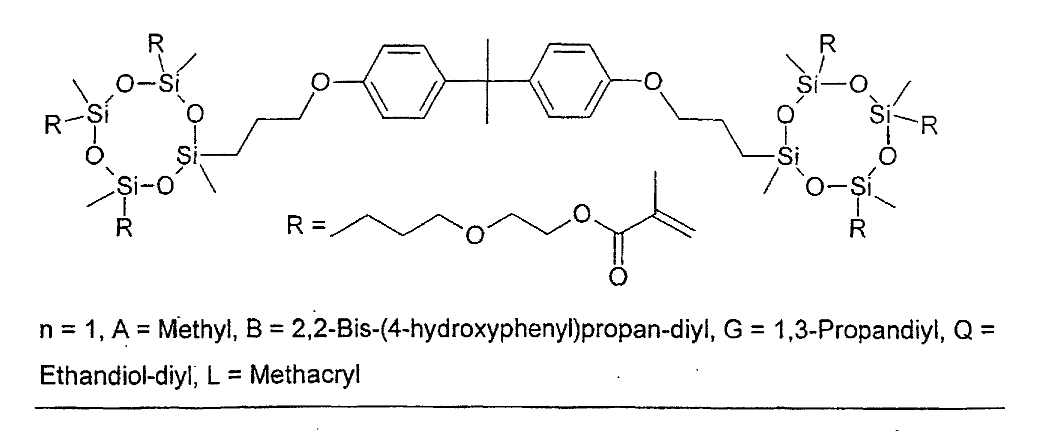

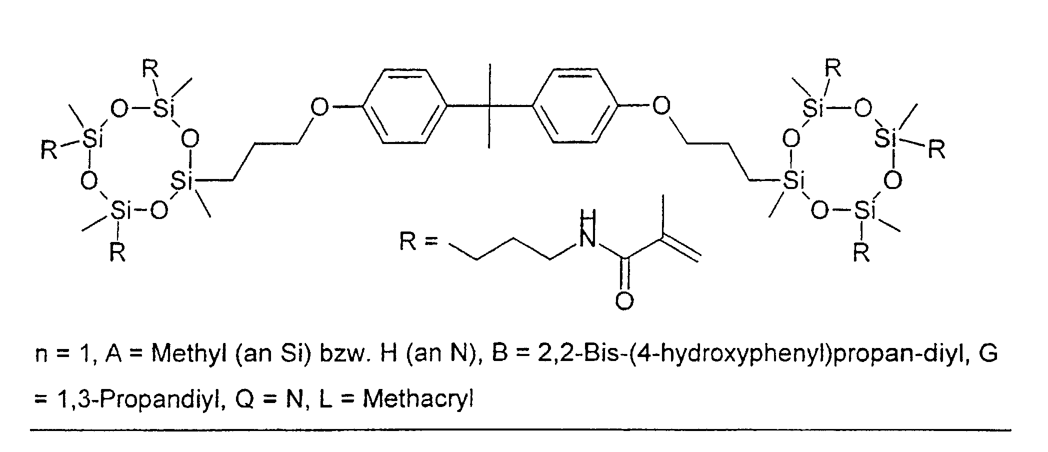

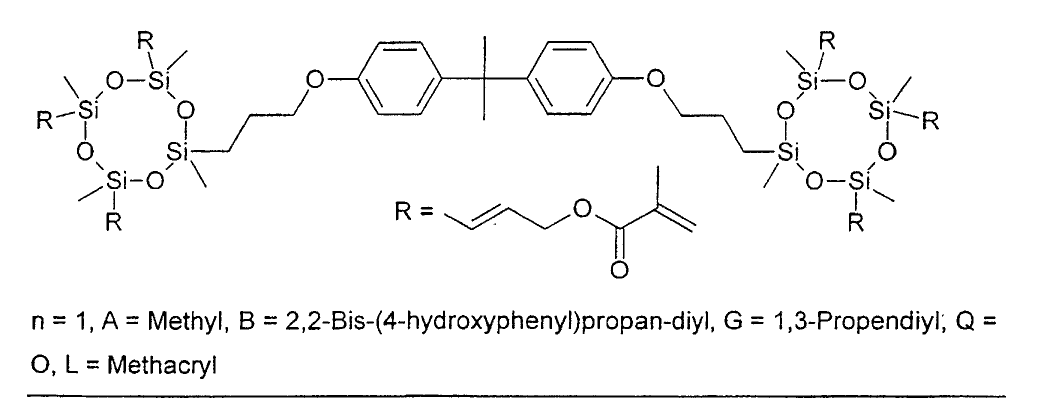

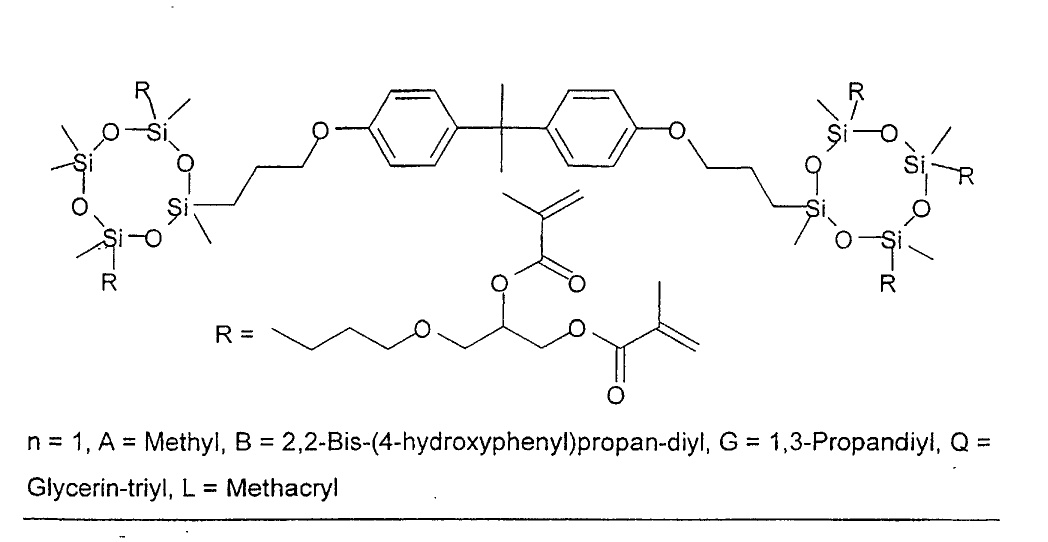

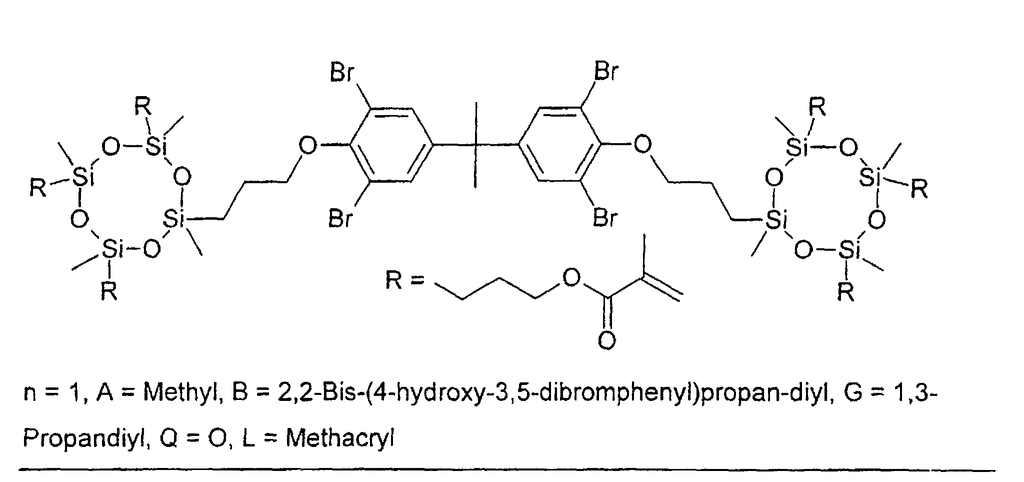

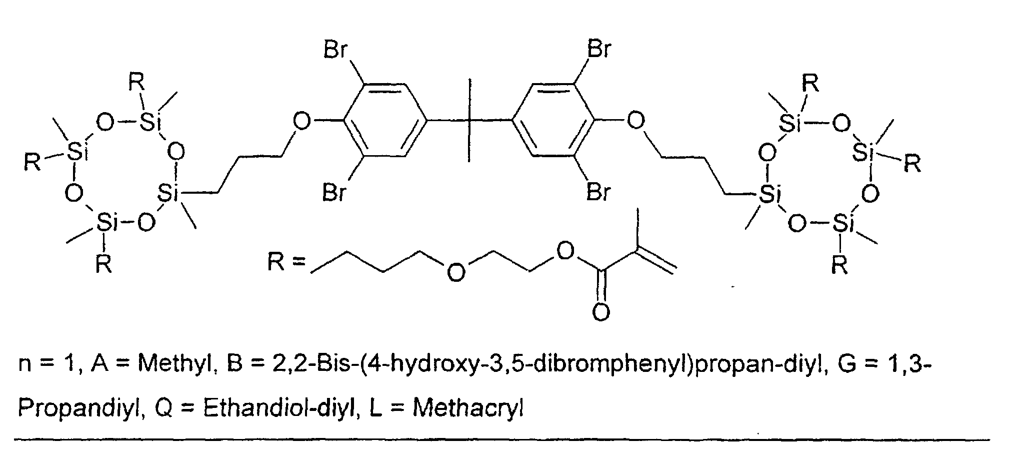

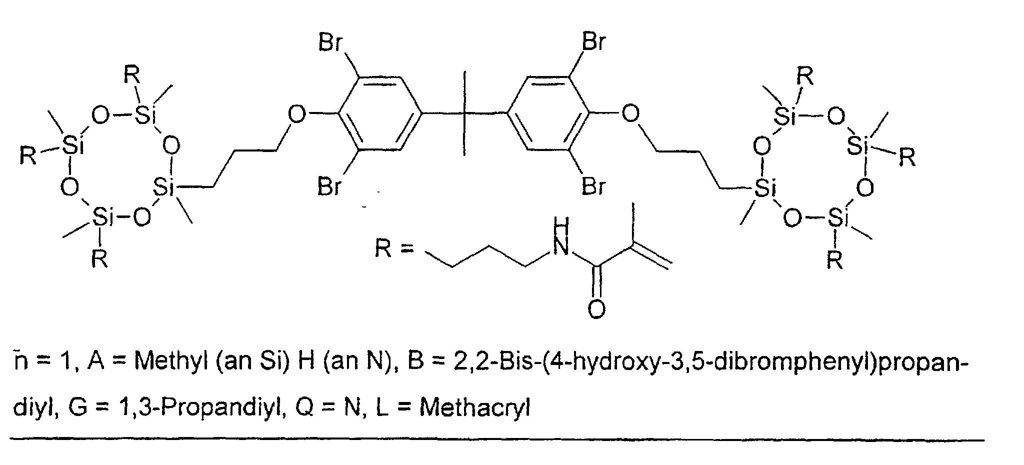

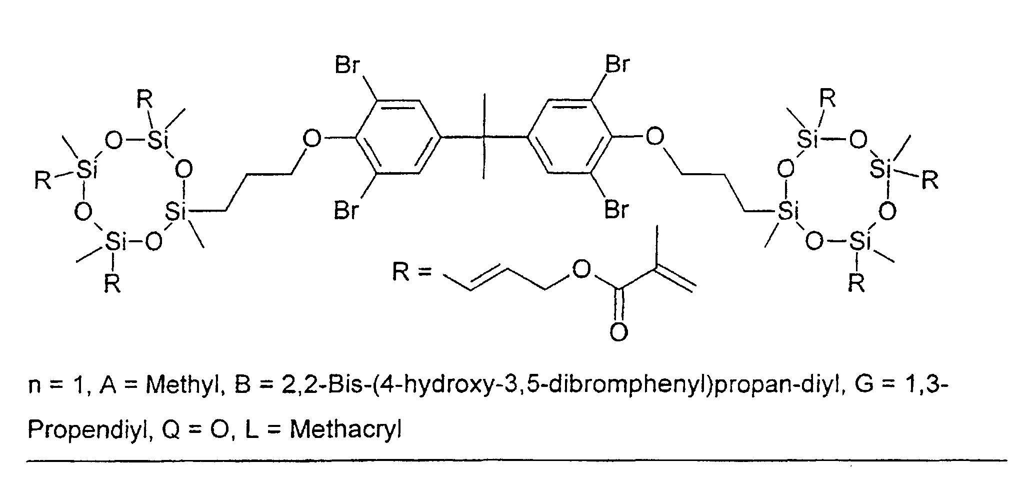

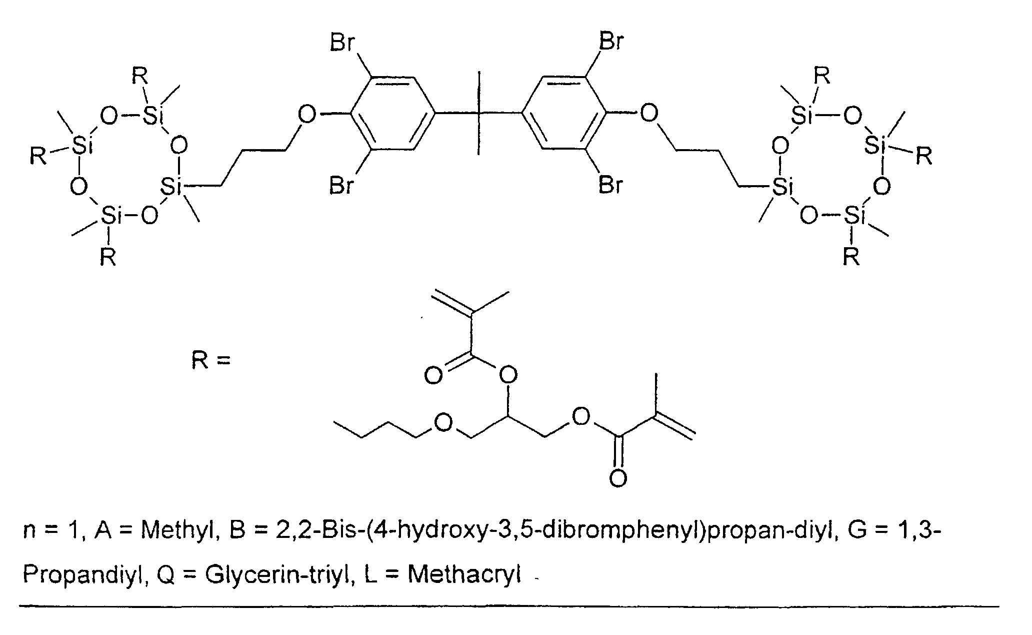

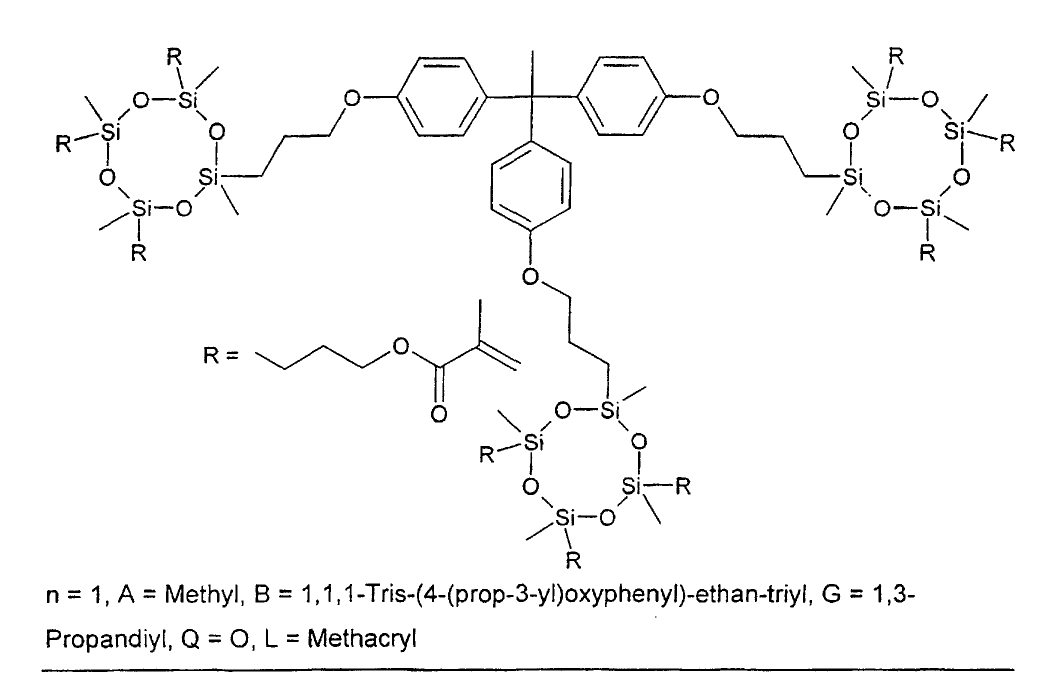

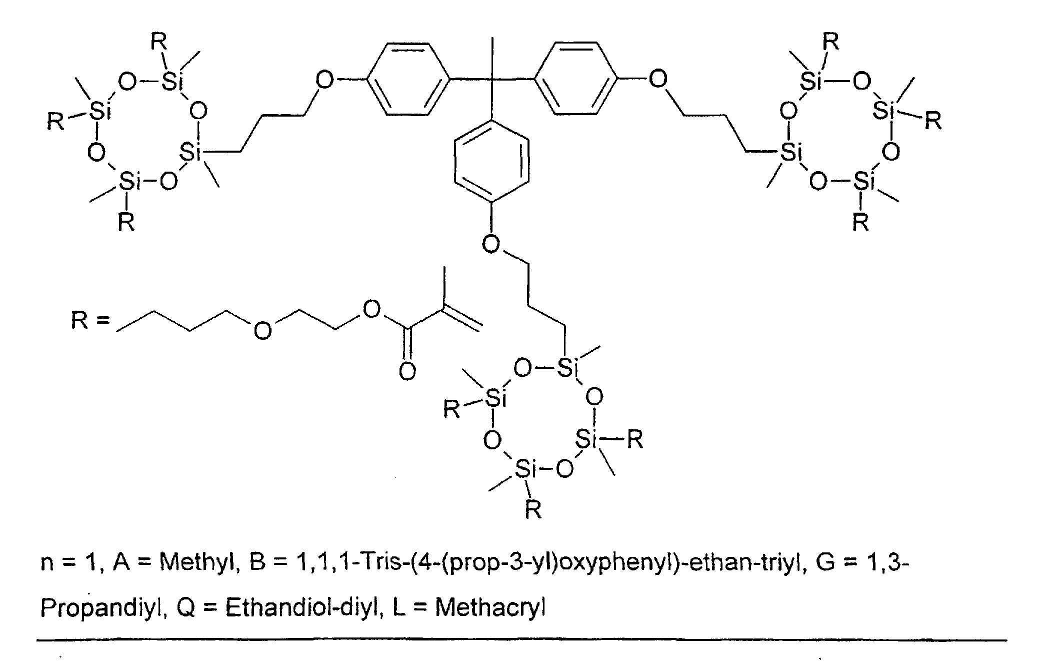

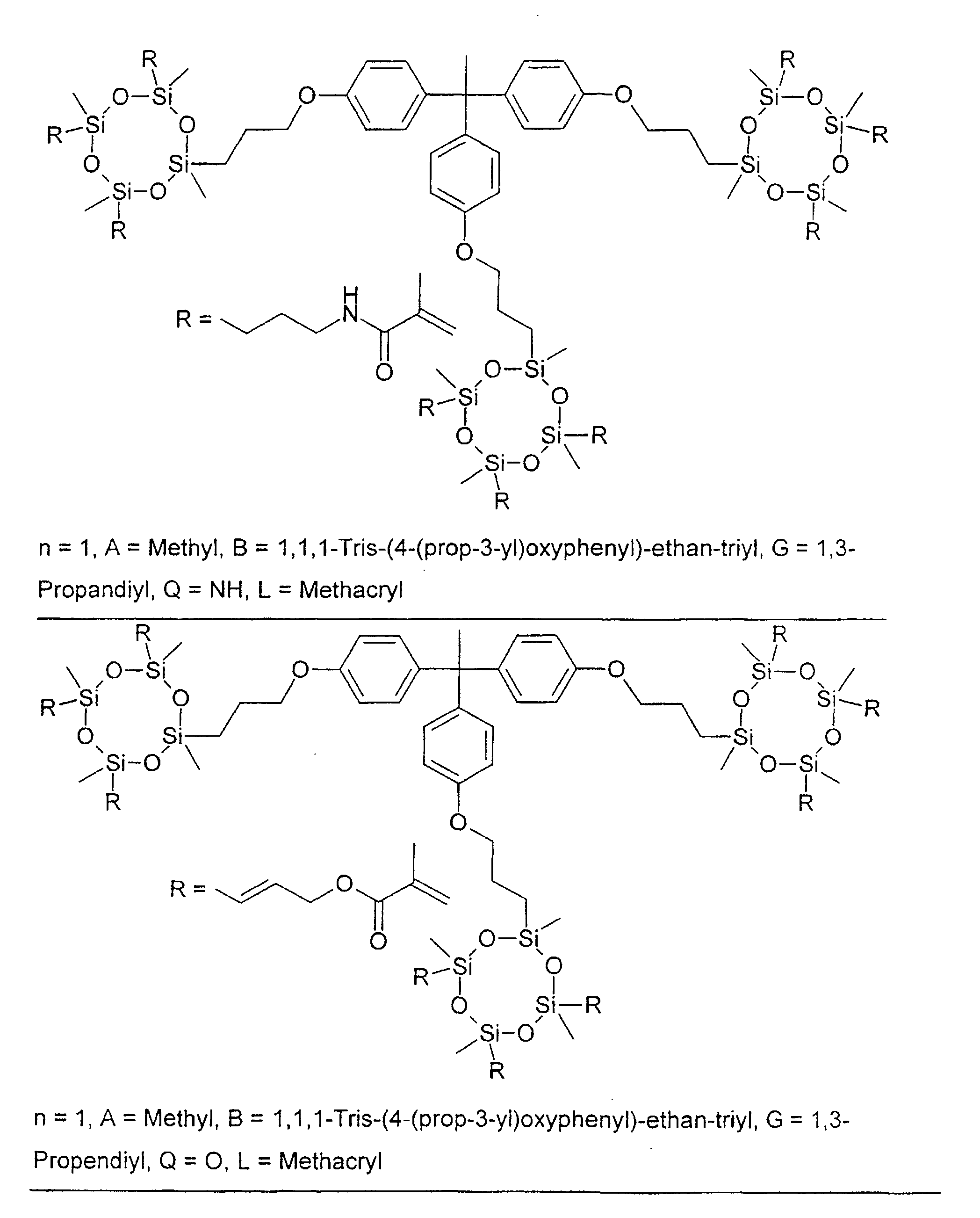

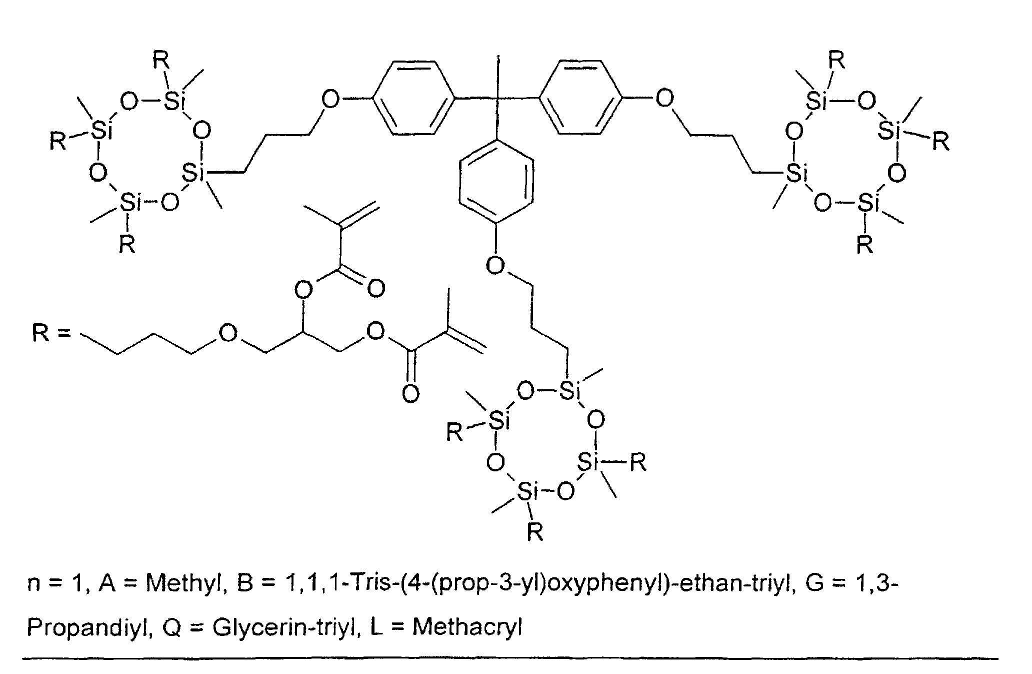

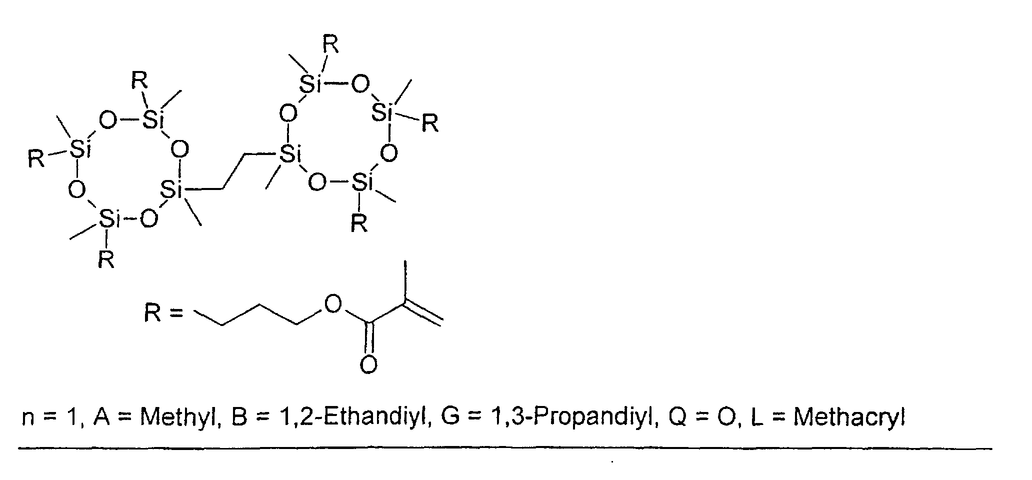

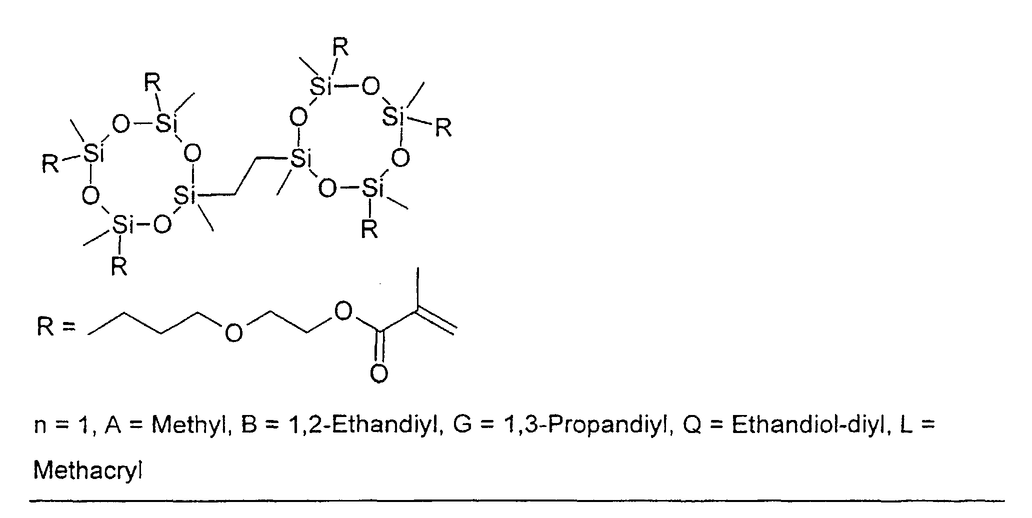

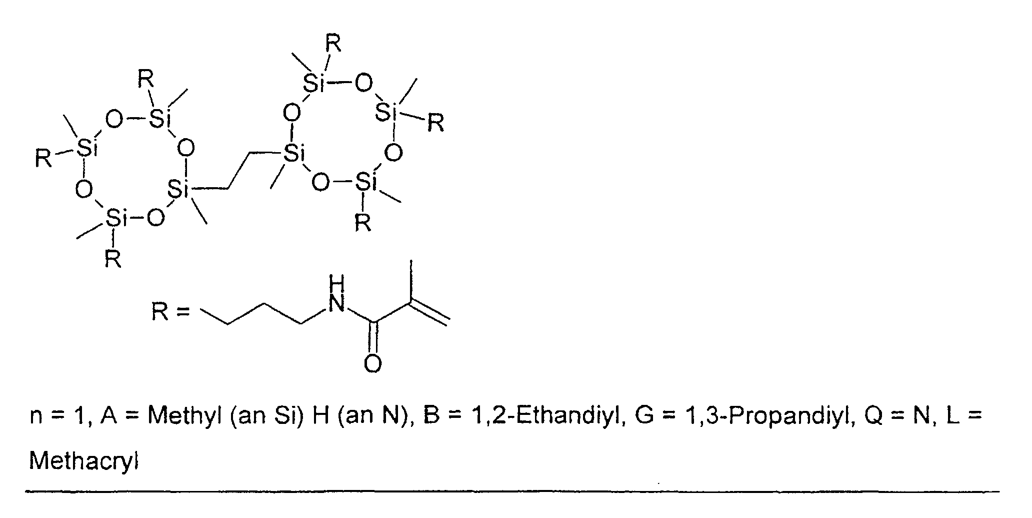

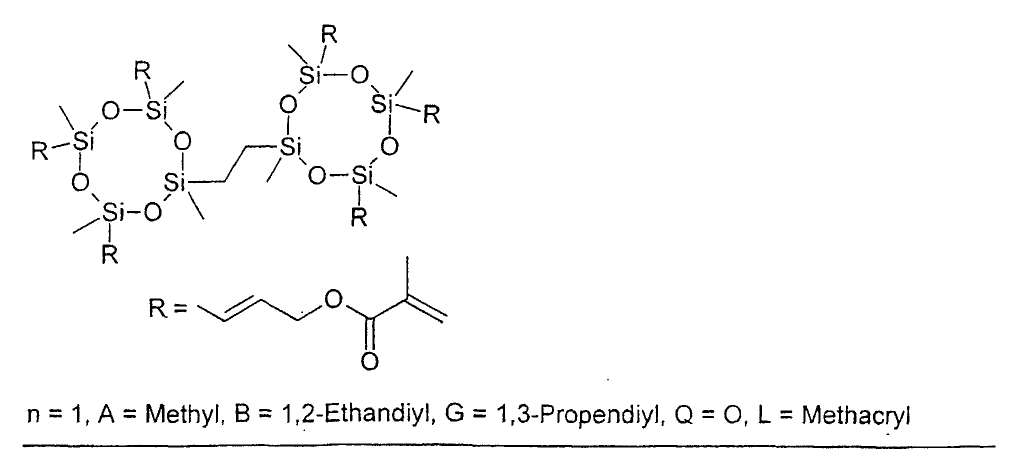

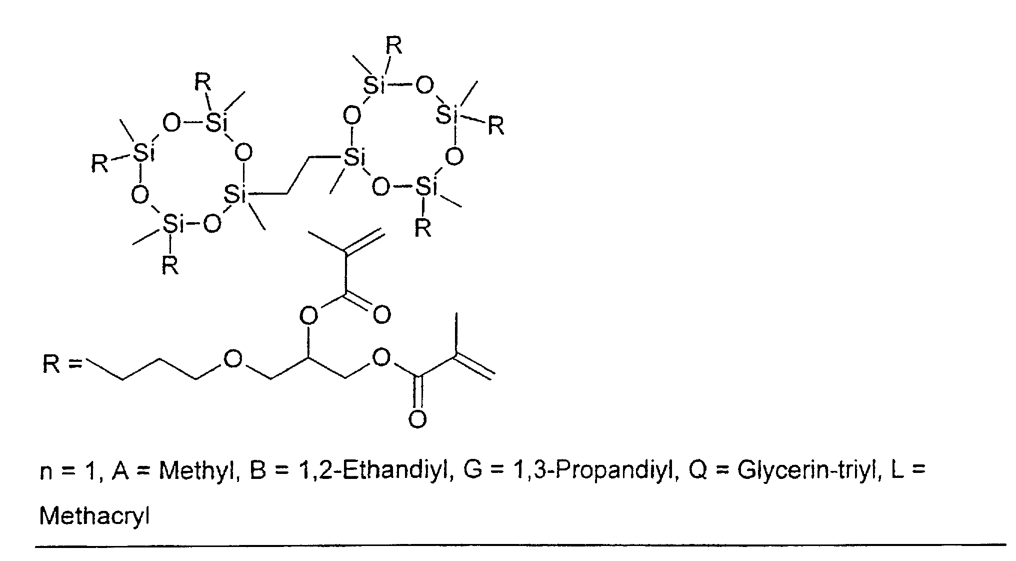

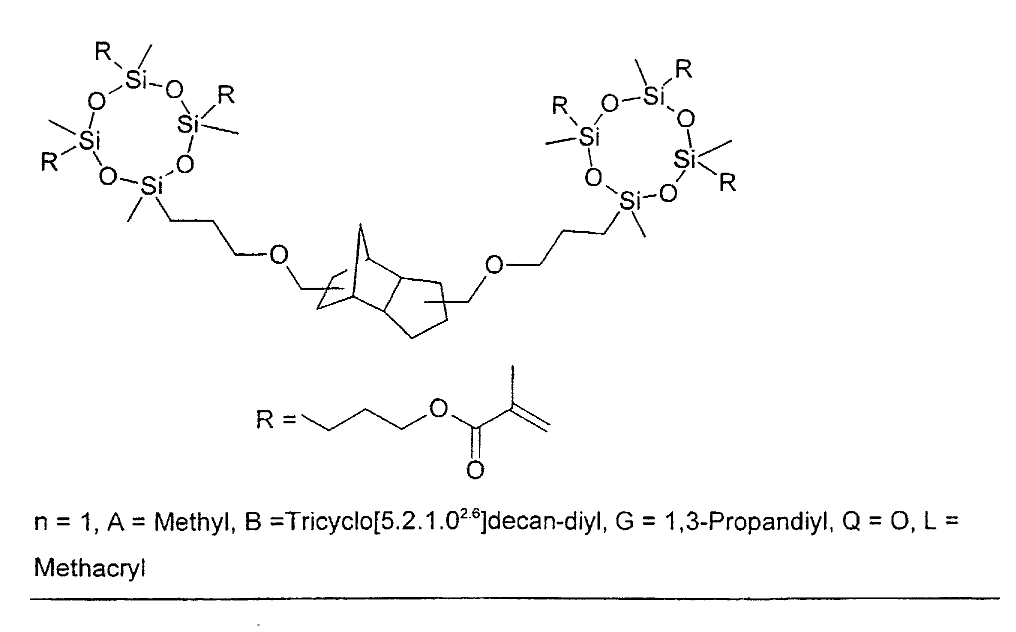

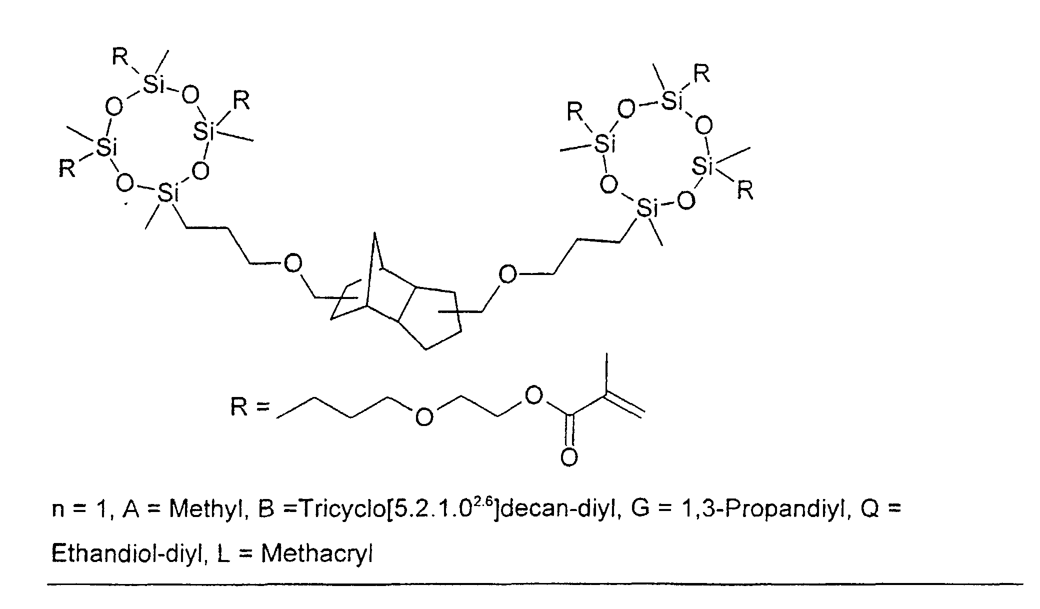

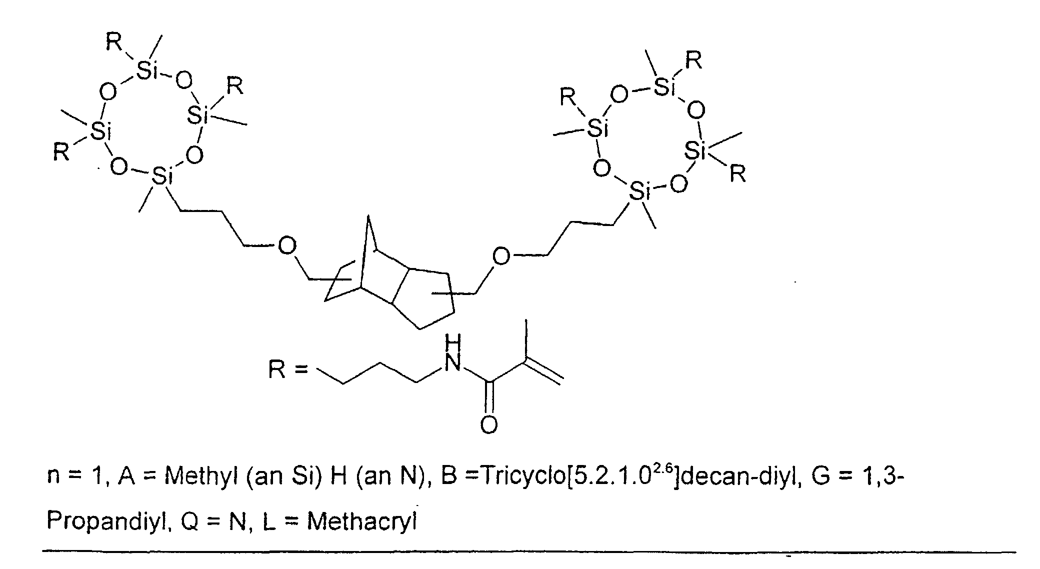

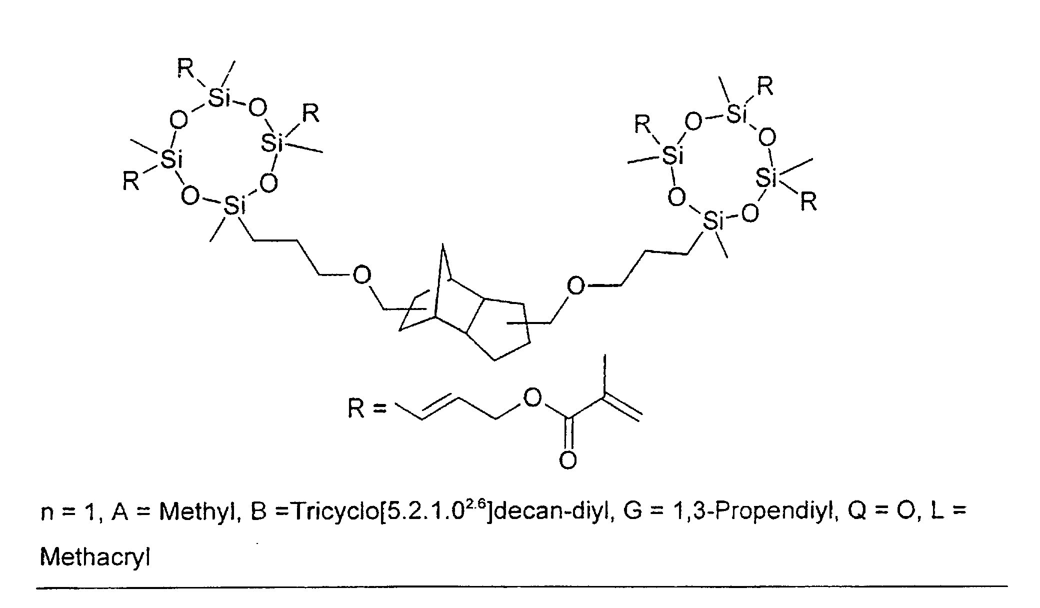

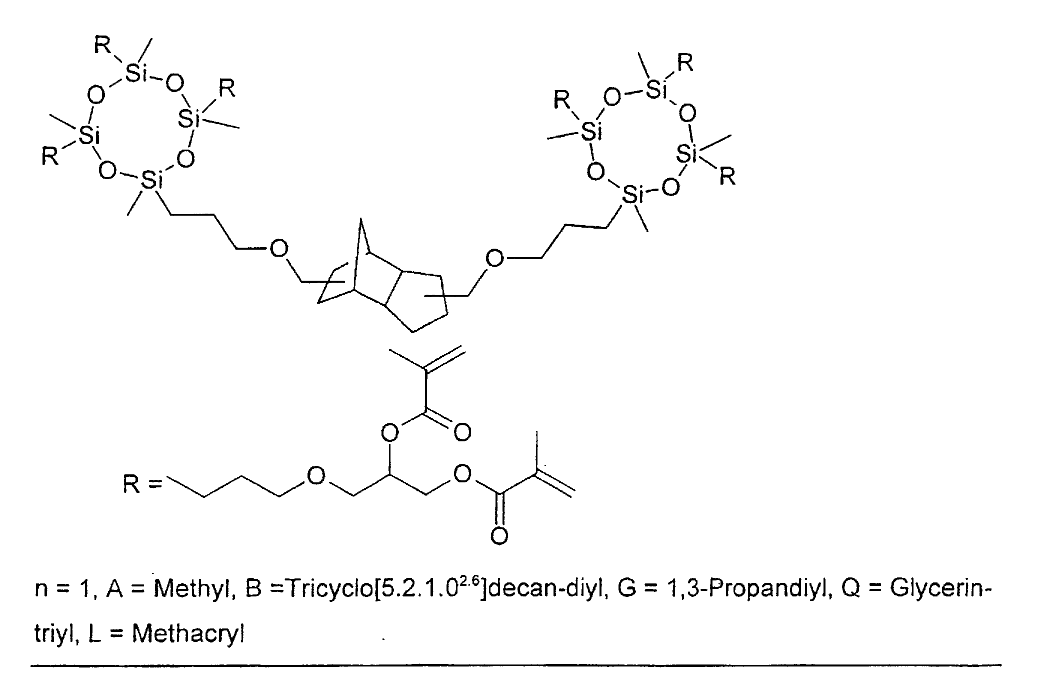

Besonders bevorzugt sind auch diejenigen Verbindungen die statt Methacryl- ganz oder teilweise Acryl-Gruppen tragen.

Ferner wurde gefunden, daß auch Verbindungen der folgenden allgemeinen Formel (II) die oben beschriebene Aufgabe zu lösen vermögen. 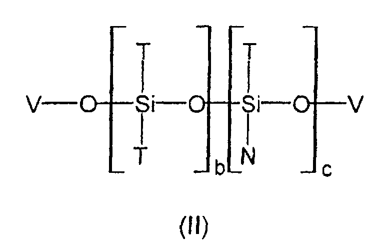 worin bedeuten:
- T=: unabhängig voneinander H oder C₁-C₁₀-Alk(en)yl, bevorzugt Methyl, Ethyl, Propyl, Butyl, Vinyl, Ethinyl, Allyl, C₃-C₁₀-Cycloalk(en)yl, bevorzugt Cyclopentyl, Cyclohexyl, Cyclopentadienyl, Cyclohexenyl, C₆-C₁₂-Aryl, bevorzugt Phenyl, Tolyl, Xylyl, oder C₈-C₁₈-Alkaryl, bevorzugt Phenylethylenyl;
- N =: eine polymerisierbare Gruppe R¹-R²-R³;
- b =: 0 bis 500, bevorzugt 0 bis 100, wobei der Anteil b höchstens 50% der Wiederholungseinheiten (b+c), bevorzugt aber 25% oder weniger betragen darf;
- c =: 1 bis 1000, bevorzugt 1 bis 500;
- R¹ =: C₁-C₁₀-Alk(en)ylen, bevorzugt Ethylen, Methylethylen, Propylen, Butylen, Hexylen, Ethenylen, Propenylen;
- R² =: O, N-T oder ein zwei- oder mehrfach-radikalischer linearer, verzweigter oder cyclischer Alkohol-, Amin- oder Aminoalkohol-Rest mit 2 bis 10 C-Atomen, bevorzugt Ethandiol-diyl, Glycerin-triyl, Trimethylolpropan-triyl, Pentaerythrittetryl;
- R³ =: ein eine C=C-Doppelbindung enthaltender organischer Rest mit 3 bis 10 C-Atomen, bevorzugt Acryl oder Methacryl;
- V=: SiMe₂T, SiEt₂T, SiMePhT, SiPh₂T.

Substanzen gemäß Formel (II) sind bekannt und die Synthese einiger beispielhafter Vertreter ist beispielsweise in B. Marciniel; Appl. Organomet. Chem. (1997), 11, 843-849 beschrieben. Ihre Eignung zur Verwendung in Dentalmassen ist bislang jedoch nicht offenbart worden.

Die funktionalisierten Siloxanmonomere der Formeln (I) und (II) können entweder jeweils alleine, als Mischung miteinander oder unter Zusatz von üblichen Monomeren verwendet werden.

Co-Monomere sind mindestens einfach ethylenisch ungesättigt. Bevorzugt verwendete ethylenisch ungesättigte Co-Monomere sind Acrylate oder Methacrylate. Geeignet sind allgemein ein- und mehrfunktionelle (Meth)acrylatmonomere. Typische Vertreter dieser Verbindungsklasse (DE-A-43 28 960) sind Alkyl(meth)acrylate, einschließlich der Cycloalkyl(meth)acrylate, Aralkyl(meth)acrylate und 2-Hydroxyalkyl(meth)acrylate, beispielsweise Hydroxypropylmethacrylat, Hydroxyethylmethacrylat, Isobornylacrylat, Isobornylmethacrylat, Butylglycolmethacrylat, Acetylglykolmethacrylat, Triethylenglycoldimethacrylat, Polyethylenglycoldimethacrylat, 2-Phenyl-ethylmethacrylat, 2-Ethylhexylmethacrylat, Cyclohexylmethacrylat, Laurylmethacrylat und Hexandioldi(meth)acrylat. Verwendet werden können auch langkettige Monomere der US-A-3 066 112 auf der Basis von Bisphenol A und Glycidylmethacrylat oder deren durch Addition von Isocyanaten entstandenen Derivate. Geeignet sind auch Verbindungen des Typs Bisphenyl-Adiethyloxy(meth)acrylat und Bisphenol-A-dipropyloxy(meth)acrylat. Weiterhin Verwendung finden können die oligoethoxylierten und oligopropoxylierten Bisphenol-A-diacryl und -dimethacrylsäureester. Gut geeignet sind außerdem die in der DE-C-28 16 823 genannten Diacryl- und Dimethacrylsäureester des Bis(hydroxymethyl)-tricyclo[5.2.1.0^{2,6}]-decans und die Diacryl- und Dimethacrylsäureester der mit 1 bis 3 Ethylenoxid- und/oder Propylenoxideinheiten verlängerten Verbindungen des Bis(hydroxymethyl)-tricyclo[5.2.1.0^{2,6}]-decans.

Die erfindungsgemäßen Dentalmassen enthalten folgende Komponenten:
i) 5 bis 70, bevorzugt 5 bis 15 Massenteile Monomere gemäß Formel (I),
ii) 0 bis 15, bevorzugt 5 bis 15 Massenteile Monomere gemäß Formel (II),
iii) 0 bis 50, bevorzugt 3 bis 10 Massenteile Co-Monomere,
iv) 20 bis 90, bevorzugt 70 bis 85 Massenteile Füllstoffe,
v) 0,001 - 5, bevorzugt 0,1 bis 2 Massenteile Initiatoren,
vi) 0 bis 20 Massenteile Hilfsstoffe,

Die Füllstoffe der hier offenbarten Massen (Komponente (iv)) sind in der Regel anorganische Füllstoffe. Beispielhaft genannt seien Quarz, gemahlene Gläser, Kieselgele sowie pyrogene Kieselsäuren und Fällungskieselsäuren oder deren Granulate. Bevorzugt werden röntgenopake Füllstoffe, zumindest teilweise, mit eingesetzt. Diese können beispielsweise röntgenopake Gläser sein, also Gläser, welche beispielsweise Strontium, Barium oder Lanthan enthalten (z.B. nach US-A-3 971 754) oder ein Teil der Füllkörper besteht aus einem röntgenopaken Zusatz, wie beispielsweise Yttriumtrifluorid, Strontiumhexafluorozirkonat oder Fluoriden der Selten-Erdmetalle (z.B. nach EP-A-0 238 025). Zum besseren Einbau in die Polymermatrix ist es von Vorteil, die anorganischen Füllstoffe zu hydrophobieren. Übliche Hydrophobierungsmittel sind Silane, beispielsweise Trimethoxymethacryloyloxypropylsilan oder Trimethoxyglycidylsilan.

Die Füllkörper haben vorzugsweise eine mittlere Kornverteilung <20µm und insbesondere <5 µm sowie eine obere Korngrenze von 150, vorzugsweise 70 µm und insbesondere 25 µm.
Besonders bevorzugt werden Gemische von 5 bis 25 Gew.-% Füllstoffe mit einer mittleren Korngröße von 0,02 bis 0,06 µm und 65 bis 85 Gew.-% Füllkörper mit einer mittleren Korngröße von 1 bis 5 µm verwendet.

Als Initiatoren (Komponente (v)) der erfindungsgemäßen Massen werden solche Systeme verwendet, die in einem geeigneten Zeitraum Radikale zu bilden vermögen.

Bei einkomponentigen Massen werden hierfür Photoinitiatoren eingesetzt, die durch Bestrahlung mit UV- oder sichtbarem Licht die Polymerisationsreaktion auslösen können.

Vertreter solcher Photoinitiatoren sind beispielsweise Benzoinalkylether, Benzilketale, Acylphosphinoxide oder aliphatische und aromatische 1,2-Diketonverbindungen, beispielsweise Campherchinon, wobei die Lichtpolymerisation durch Zusatz von Aktivatoren, wie tertiären Aminen oder organischen Phosphiten, in an sich bekannter Weise beschleunigt werden kann.

Geeignete Initiatorsysteme zur Auslösung der Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor ihrer Anwendung miteinander homogen vermischt.

Geeignete Hilfsstoffe gemäß Komponente (vi) können beispielsweise üblicherweise auf dem Dentalgebiet eingesetzte Stabilisatoren, Pigmente oder Verdünnungsmittel sein.

Die Herstellung der hier offenbarten Massen erfolgt vorzugsweise so, daß die flüssigen Bestandteile miteinander gemischt werden, die Initiatoren, sofern sie nicht flüssig sind, darin durch Rühren eingelöst werden und anschließend die Füllstoffe zugegeben werden und durch Kneten gut homogenisiert wird.

Zweikomponentige Zubereitungen, deren Aushärtung durch Redox-Mechanismen erfolgt, werden so formuliert, daß die wesentlichen Bestandteile des Redox-Initiierungssystems getrennt in je einem Teil der zweikomponentigen Zubereitung eingebracht werden. Die Aufteilung der Bestandteile der Gesamtzubereitung richtet sich nach den jeweiligen Lagerbeständigkeiten und dem angestrebten Mischungsverhältnis.

Die polymerisierbaren Massen zeichnen sich durch einen hohen Füllstoffanteil und damit verbundener hoher Festigkeit bei gleichzeitig guter Verarbeitbarkeit aus.

Die erfindungsgemäßen Massen eigenen sich insbesondere als Werkstoffe für dentale Zwecke, beispielsweise zur Herstellung von Kunststoffzähnen oder Provisorien, als Beschichtungsmittel, zum Verkleben von Substraten sowie als dentale Füllungsmaterialien. Als Dentalmassen eignen sich die erfindungsgemäßen Massen insbesondere zur Herstellung von polymerisierbaren Füllungsmaterialien, Befestigungszementen, Inlays, Onlays, Verblendschalen, sowie provisorischen Kronen- und Brückenmaterialien.

Im folgenden wird die Erfindung durch Beispiele näher erläutert:

### Herstellungsbeispiel

### Herstellung von 1,3,5,7 Tetramethyl-1,3,5,7-tetrakis-(3-methacryloxypropyl)-cyclotetrasiloxan

3,6 g 1,3,5,7-Tetramethylcyclotetrasiloxan, 38,2 g Allylmethacrylat und 110 ml Toluol werden mit Karstedt-Katalysator (3 - 3,5% Pt, 300 ppm Pt, ABCR) 24 Stunden gerührt. Mittels IR wird der Umsatz geprüft, bei noch vorhandener Si-H Bande bei etwa 2100 cm⁻¹ nachgerührt und anschließend im Vakuum das Lösungsmittel abgezogen.
Ausbeute 46,7 g = 90%.

### Anwendungsbeispiele 1 - 6, Vergleichsbeispiel

### Herstellung von Dentalmassen mit und ohne Verwendung der Monomere vom Typ (I) und (II)

Für die Herstellung der erfindungsgemäßen Dentalzubereitungen wurde das gemäß dem Herstellungsbeispiel hergestellte Tetrakismethacrylcyclotetrasiloxan verwendet. Für das Vegleichsbeispiel wurde diese Komponente weggelassen.

In einem 100 ml-Laborkneter wurden die pastenförmigen Zubereitungen gemäß den Anwendungsbeispielen 1 bis 6 und gemäß dem Vergleichsbeispiel, deren Zusammensetzungen in Tabelle 1 beschrieben sind, hergestellt.

Die Zubereitungen wurden hinsichtlich Druckfestigkeit sowie Biegefestigkeit und E-Modul gemäß DIN ISO 4049 charakterisiert.

Die Herstellung der Prüfkörper erfolgte durch 40 Sekunden Bestrahlung der in Formen eingebrachten pastenförmigen Zubereitungen mit dem Lichtgerät Elipar II der Firma ESPE Dental AG, Deutschland.

Die Prüfkörper wurden nach der Entformung für einen Zeitraum von 24 Stunden in deionisiertes Wasser bei 36°C eingelagert und danach die mechanischen Eigenschaften ermittelt.

Durch Bestimmung der Dichten der pastenförmigen Zubereitungen und der ausgehärteten Massen nach der Auftriebsmethode wurde der bei der radikalischen Polymerisation eintretende Volumenschrumpf festgestellt.

Eine Zusammenstellung der an den ausgehärteten Zubereitungen gemäß den Anwendungsbeispielen 1 bis 6 und gemäß dem Vergleichsbeispiel ermittelten Eigenschaftswerten enthält Tabelle 2.

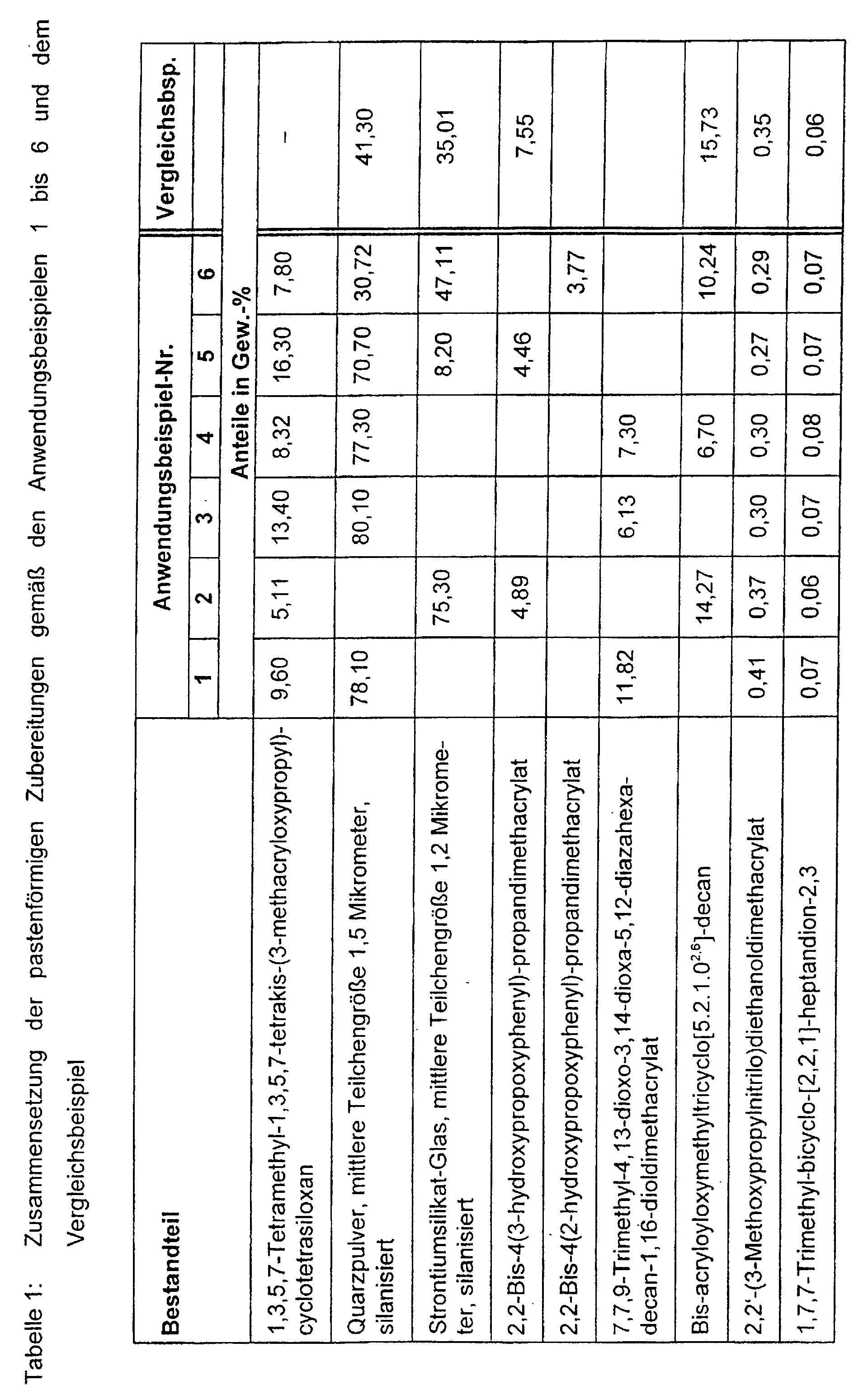

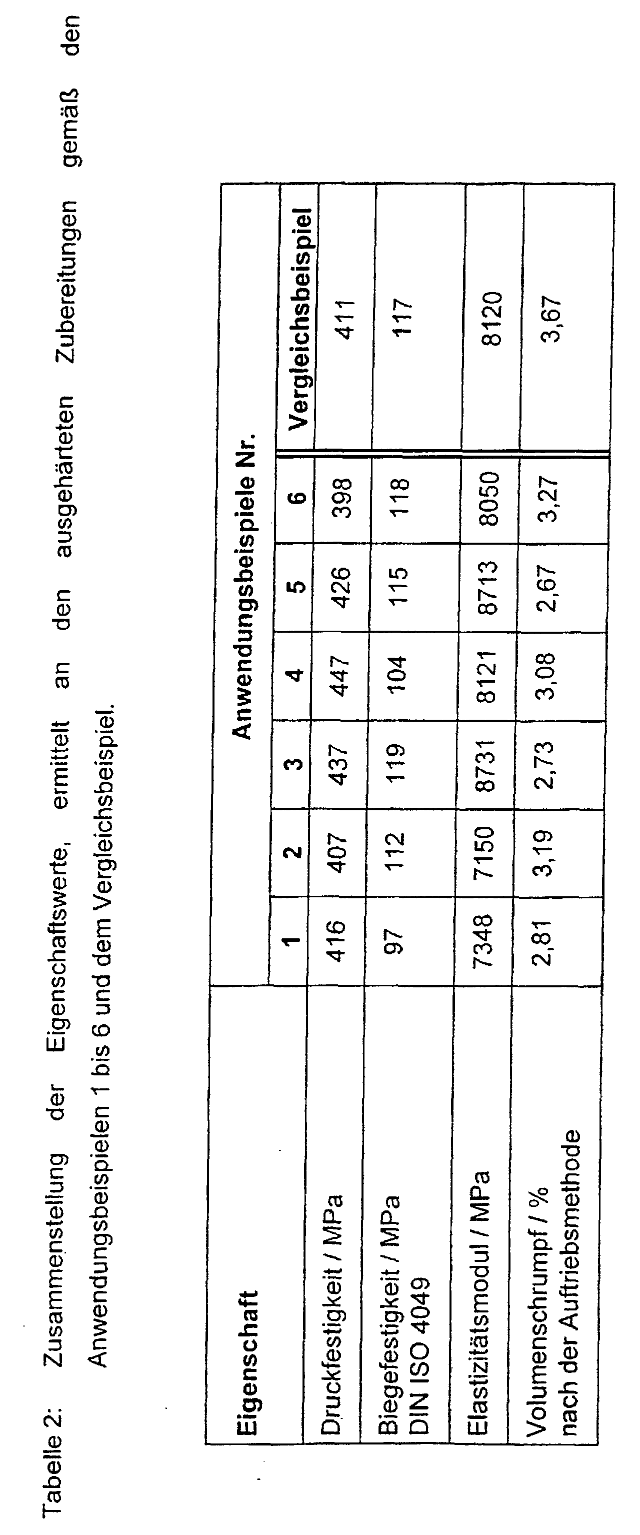

## Patentansprüche

1. Cyclische Siloxanverbindungen der folgenden allgemeinen Formel (I): 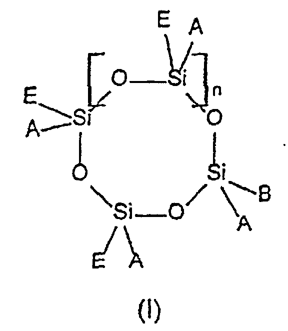 worin bedeuten:
n = 0,1,2,3,4,5,6,7,8,9,10 bevorzugt 1,2,3,4,5;
A= H oder C₁-C₁₅-Alk(en)yl, C₃-C₁₅-Cycloalk(en)yl, C₆-C₁₂-Aryl, C₈-C₁₈-Alkaryl, wobei in den genannten Resten jeweils ein oder mehrere C-Atome durch O, C=O, O(C=O), SiR₂ und / oder NR ersetzt sein können, wobei R ein aliphatischer Rest mit 1 bis 7 C-Atomen ist, bei dem ein oder mehrere C-Atome durch O, C=O und / oder O(C=O) ersetzt sein können;
B = E oder ein linearer, verzweigter oder polycyclischer aliphatische oder aromatische Gruppen enthaltender Kohlenwasserstoffrest ist, der 2 bis 10 der oben definierten Cyclosiloxanreste, abzüglich B, miteinander verknüpft und 2 bis 50 C-Atome und zusätzlich 0 bis 30 andere Atome aus der Gruppe O, N, S, P, Si, Cl, F, Br, I enthält, und an welchem entsprechend 1 bis 9, vorzugsweise 1 bis 4 der oben definierten Cyclosiloxanreste, abzüglich B, hängen;
E = A oder eine polymerisierbare Gruppe G-Q-L, wobei durchschnittlich bis zu 25% oder weniger der Gruppen E Vertretern von A entsprechen;
G = C₁-C₁₀-Alk(en)ylen;
Q = O, N-A oder ein zwei- oder mehrfach-radikalischer linearer, verzweigter oder cyclischer Alkohol-, Amin- oder Aminoalkohol-Rest mit 2 bis 10 C-Atomen;
L = ein eine C=C-Doppelbindung enthaltender organischer Rest mit 2 bis 10 C-Atomen;
und mit der Maßgabe, daß in (I) keine annelierten Siloxanringsysteme vorkommen können.

2. Dentalmasse enthaltend
i) 5 bis 70 Massenteile Monomere gemäß Formel (I),
ii) 0 bis 15 Massenteile Monomere gemäß Formel (II),
iii) 0 bis 50 Massenteile Co-Monomere,
iv) 20 bis 90 Massenteile Füllstoffe,
v) 0,001 bis 5 Massenteile Initiatoren
vi) 0 bis 20 Massenteile Hilfsstoffe,
wobei Komponente ii) Verbindungen der folgenden allgemeinen Formel (II) sind: 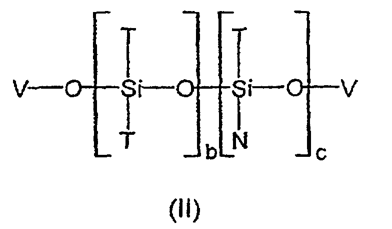 worin bedeuten:
T = unabhängig voneinander H oder C₁-C₁₀-Alk(en)yl, C₃-C₁₀-Cycloalk(en)yl, C₆-C₁₂-Aryl oder C₈-C₁₈-Alkaryl;
N = eine polymerisierbare Gruppe R¹-R²-R³;
b = 0 bis 500, wobei der Anteil b höchstens 50% der Wiederholungseinheiten (b+c) betragen darf;
c = 1 bis 1000;
R¹ = C₁-C₁₀-Alk(en)ylen;
R² = O, N-T oder ein zwei- oder mehrfach-radikalischer linearer, verzweigter oder cyclischer Alkohol-, Amin- oder Aminoalkohol-Rest mit 2 bis 10 C-Atomen;
R³ = ein eine C=C-Doppelbindung enthaltender organischer Rest mit 3 bis 10 C-Atomen;
V= SiMe₂T, SiEt₂T, SiMePhT, SiPh₂T.

3. Dentalmasse nach Anspruch 2, in der die Variablen der Verbindungen (II) wie folgt definiert sind:
T = Methyl, Ethyl, Propyl, Butyl, Vinyl, Ethinyl, Allyl, Cyclopentyl, Cyclohexyl, Cyclopentadienyl, Cyclohexenyl, Phenyl, Tolyl, Xylyl, Phenylethylenyl;
b = 0 bis 100, wobei der Anteil b 25% oder weniger der Wiederholungseinheiten (b+c) betragen darf;
c = 1 bis 500;
R¹ = Ethylen, Methylethylen, Propylen, Butylen, Hexylen, Ethenylen, Propenylen;
R² = Ethandiol-diyl, Glycerin-triyl, Trimethylolpropan-triyl, Pentaerythrit-tetryl;
R³ = Acryl oder Methacryl;

4. Verwendung einer Dentalmasse nach Anspruch 2 zur Herstellung von polymerisierbaren Füllmaterialien, Befestigungszementen, Inlays, Onlays, Verblendschalen sowie provisorischen Kronen und Brückenmaterialien.

5. Verfahren zur Herstellung der Dentalmasse nach Anspruch 2, **dadurch gekennzeichnet, daß** man die Komponenten (i) bzw. (ii), gegebenenfalls (iii), (iv), (v) und gegebenenfalls (vi) miteinander vermischt.

## Claims

1. Cyclic siloxane compounds of the following general formula (I): 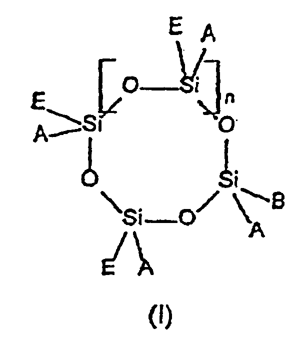 in which:
n = 0, 1, 2, 3, 4, 5, 6, 7, 8, 9, 10 preferably 1, 2, 3, 4, 5;
A = H or C₁-C₁₅ alk(en)yl, C₃-C₁₅ cycloalk(en)yl, C₆-C₁₂ aryl, C₈-C₁₈ alkaryl, where, in the said radicals, in each case one or more C atoms can be replaced by O, C=O, O(C=O), SiR₂ and/or NR, R being an aliphatic radical with 1 to 7 C atoms, in which one or more C atoms can be replaced by O, C=O, and/or O(C=O);
B = E or a linear, branched or polycyclic aliphatic- or aromatic-groups-containing hydrocarbon radical which links 2 to 10 of the cyclosiloxane radicals defined above, less B, to one another and contains 2 to 50 C atoms and additionally 0 to 30 other atoms from the group O, N, S, P, Si, Cl, F, Br, I and from which correspondingly 1 to 9, preferably 1 to 4 of the above defined cyclosiloxane radicals, less B, are pending;
E = A or a polymerizable group G-Q-L, where on average up to 25% or less of the groups E correspond to representatives of A;
G = C₁₋₁₀ alk(en)ylene;
Q = O, N-A or a di- or polyvalent linear, branched or cyclic alcohol, amine or aminoalcohol radical with 2 to 10 C atoms;
L = an organic radical, containing a C=C double bond, with 2 to 10 C atoms;
and with the proviso that no annelated siloxane ring systems can occur in (I).

2. Dental composition containing
i) 5 to 70 parts by mass monomers according to formula (I),
ii) 0 to 15 parts by mass monomers according to formula (II),
iii) 0 to 50 parts by mass co-monomers,
iv) 20 to 90 parts by mass fillers,
v) 0.001-5 parts by mass initiators,
vi) 0 to 20 parts by mass auxiliaries,
where component ii) are compounds of the following general formula (II): 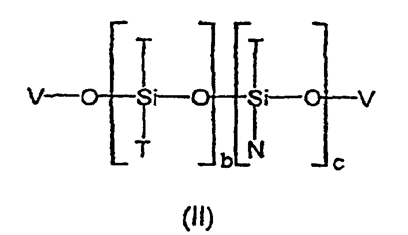 in which:
T = independently of each other H or C₁-C₁₀ alk(en)yl, C₃-C₁₀ cycloalk(en)yl, C₆-C₁₂ aryl or C₈-C₁₈ alkaryl;
N = a polymerizable group R¹-R²-R^{3;}
b = 0 to 500, where the proportion b may be at most 50% of the repeat units (b+c);
c= 1 to 1000;
R¹ = C₁-C₁₀ alk(en)ylene;
R² = O, N-T or a di- or polyvalent linear, branched or cyclic alcohol, amine or aminoalcohol radical with 2 to 10 C atoms;
R³ = an organic radical, containing a C=C double bond, with 3 to 10 C atoms;
V = SiMe₂T, SiEt₂T, SiMePhT, SiPh₂T.

3. Dental composition according to claim 2, in which the variables of the compounds (II) are defined as follows:
T = methyl, ethyl, propyl, butyl, vinyl, ethinyl, allyl, cyclopentyl, cyclohexyl, cyclopentadienyl, cyclohexenyl, phenyl, tolyl, xylyl, phenylethylenyl;
b = 0 to 100, whereby the proportion b may be 25% or less of the repeat units (b+c);
c = 1 to 500;
R¹ = ethylene, methylethylene, propylene, butylene, hexylene, ethenylene, propenylene;
R² = ethanediol-diyl, glycerol-triyl, trimethylolpropane-triyl, pentaerythritol-tetryl;
R³ = acryl or methacryl;

4. Use of a dental composition according to claim 2 for the preparation of polymerizable filling materials, fixing cements, inlays, onlays, facing shells and temporary crowns and bridge materials.

5. Method of preparing the dental composition according to claim 2, **characterized in that** the components (i) and (ii), optionally (iii), (iv), (v) and optionally (vi) are mixed with one another.

## Revendications

1. Composés cycliques de siloxanes ayant la formule générale (I) suivante : 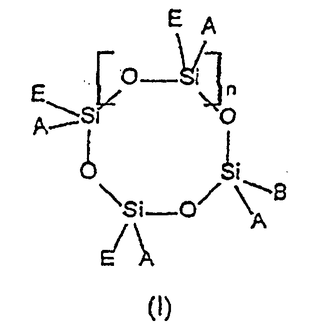 dans laquelle :
n = 0, 1, 2, 3, 4, 5, 6, 7, 8, 9, 10, de préférence 1, 2, 3, 4, 5 ;
A = H ou alkyle/alcényle en C₁-C₁₅, cycloalkyle/cyclo-alcényle en C₃-C₁₅, aryle en C₆-C₁₂, alcaryle en C₆-C₁₈, un ou plusieurs atomes de carbone pouvant être remplacés à chaque fois par O, C=O, O(C=O), SiR₂ et/ou NR dans lesdits restes, R étant un reste aliphatique ayant de 1 à 7 atomes de carbone, dans lequel un ou plusieurs atomes de carbone peuvent être remplacés par O, C=O et/ou O(C=O) ;
B = E ou un reste hydrocarboné linéaire, ramifié ou polycyclique, contenant des groupes aliphatiques ou aromatiques, qui relie de 2 à 10 des restes cyclosiloxanes définis ci-dessus, déduction faite de B, et qui contient de 2 à 50 atomes de carbone et, en outre, de 0 à 30 autres atomes choisis dans l'ensemble constitué de O, N, S, P, Si, Cl, F, Br, I, et auquel sont suspendus de façon correspondante de 1 à 9, de préférence de 1 à 4 des restes cyclosiloxanes définis ci-dessus, déduction faite de B ;
E = A ou un groupe polymérisable G-Q-L, en moyenne jusqu'à 25 % ou moins des groupes E correspondent à des exemples représentatifs de A ;
G = alkylène/alcénylène en C₁-C₁₀ ;
Q = O, N-A ou un reste alcool, aminé ou amino-alcool bi- ou multiradicalaire linéaire, ramifié ou cyclique, ayant de 2 à 10 atomes de carbone ;
L = un reste organique contenant une liaison double C=C et ayant de 2 à 10 atomes de carbone ;
et avec la condition que des systèmes cycliques de siloxanes condensés n'apparaissent pas dans (I).

2. Composition dentaire contenant
i) de 5 à 70 parties en poids de monomères répondant à la formule (I),
ii) de 0 à 15 parties en poids de monomères répondant à la formule (II),
iii) de 0 à 50 parties en poids de comonomères,
iv) de 20 à 90 parties en poids de charges,
v) de 0,001 à 5 parties en poids d'initiateurs,
vi) de 0 à 20 parties en poids d'auxiliaires,
les composants ii) étant des composés ayant la formule générale (II) suivante : 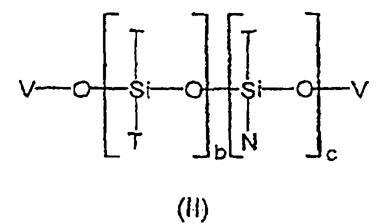 dans laquelle :
T = indépendamment les uns des autres, H ou alkyle/alcényle en C₁-C₁₀, cycloalkyle/cycloalcényle en C₃-C₁₀, aryle en C₆-C₁₂ ou alcaryle en C₈-C₁₈ ;
N = un groupe polymérisable R¹-R²-R³ ;
b = de 0 à 500, la proportion de b pouvant représenter au plus 50 % des unités répétitives (b+c) ;
c = de 1 à 1000 ;
R¹ = alkylène/alcénylène en C₁-C₁₀ ;
R² = O, N-T ou un reste alcool, amine ou amino-alcool biou multiradicalaire linéaire, ramifié ou cyclique, ayant de 2 à 10 atomes de carbone ;
R³ = un reste organique contenant une liaison double C=C et ayant de 3 à 10 atomes de carbone ;
V = SiMe₂T, SiEt₂T, SiMePhT, SiPh₂T.

3. Composition dentaire selon la revendication 2, dans laquelle les variables des composés (II) sont définies comme suit :
T = méthyle, éthyle, propyle, butyle, vinyle, éthinyle, allyle, cyclopentyle, cyclohexyle, cyclopentadiényle, cyclohexényle, phényle, tolyle, xylyle, phényléthylényle ;
b = de 0 à 100, la proportion de b pouvant représenter 25 % ou moins des unités répétitives (b+c) ;
c = de 1 à 500 ;
R¹ = éthylène, méthyléthylène, propylène, butylène, hexylène, éthénylène, propénylène ;
R² = éthanediol-diyle, glycérol-diyle, triméthylolpropanetriyle, pentaérythritol-tétryle ;
R³ = acryle ou méthacryle.

4. Utilisation d'une composition dentaire selon la revendication 2 pour la préparation de matières polymérisables d'obturation, de ciments de fixation, d'incrustations (en anglais : *inlays*), d'incrustations à recouvrement (en anglais : *onlays*), d'enveloppes de parement ainsi que de matériaux pour couronnes et bridges provisoires.

5. Procédé de préparation de la composition dentaire selon la revendication 2, **caractérisé en ce qu'**on mélange ensemble les composants (i) et (ii), le cas échéant (iii), (iv), (v) et le cas échéant (vi).
